# EUROPEAN PATENT SPECIFICATION

(11) **EP 1 367 119 B1**
(45) Date of publication and mention of the grant of the patent: **17.09.2008**
(21) Application number: 03011907.7
(22) Date of filing: 27.05.2003
(51) Int. Cl.: C12M 3/06

(54) **Methods of culturing, storing, and inducing differentiation in cells, instrument for use in the methods and method of using the instrument.**
Verfahren zur Kultur, zum Speichern und zur Induzierung von Differenzierung von Zellen und Gerät zur Verwendung in diesem Verfahren, und dazugehöriges Gebrauchsverfahren.
Procédés pour la culture, le stockage et l'induction de differentiation de cellules, instrument a cet essient et procédé d'utilisation de l'instrument.

(30) Priority: 28.05.2002 JP 2002153326; 28.05.2002 JP 2002153330; 30.10.2002 JP 2002316007; 20.11.2002 JP 2002336576; 20.11.2002 JP 2002336594; 19.03.2003 JP 2003075265
(43) Date of publication of application: 03.12.2003
(73) Proprietor: Toyo Boseki Kabushiki Kaisha, Osaka-shi, Osaka-fu (JP); Funatsu, Kazumori, Kasuga-shi, Fukuoka-ken (JP)
(72) Inventor: Yamaguchi, Tatsuya, c/o Tsuruga Inst. of Biotechn., Tsuruga-shi, Fukui-ken (JP); Ishibashi, Takuya, c/o Tsuruga Inst. of Biotechn., Tsuruga-shi, Fukui-ken (JP); Funatsu, Kazumori, Kasuga-shi, Fukuoka-ken (JP); Nakazawa, Kohji, Kitakyushu-shi, Fukuoka-ken (JP)
(74) Representative: Weber, Thomas

(56) References cited:
- EP-A- 0 475 303
- EP-A- 1 078 982
- WO-A-84/01959
- WO-A-94/25584
- US-A- 4 804 628
- US-A- 5 605 835

## Description

### BACKGROUND OF THE INVENTION

### 1. Field of the Invention

The present invention relates to a hollow fiber-possessing instrument for culturing or storing cells in hollow fibers, and to a method of using the instrument, in the field of cell culture, or tissue culture.

Moreover, the present invention relates to methods of storing, culturing, seeding, and inducing differentiation in cells while maintaining the functions of the cells .

### 2. Description of the Related Art

With a monolayer culture method, which is widely used conventionally as a general culture method for adhesive animal cells, it is difficult to maintain the functions that were originally possessed by the cells in vivo; it is well known that, although the cells survive or proliferate, the characteristic functions of the cells are rapidly lost.

For example, out of primary cultured cells, with highly differentiated primary hepatocytes, the functions thereof are particularly prone to being lost during the monolayer culture period. For example, with rat primary cultured hepatocytes, it is known that if monolayer culture is carried out in a flask, then the ammonia metabolizing function, which is an important function of hepatocytes, is usually lost within approximately 2 weeks from commencing the culture.

As one method of culturing cells for reconstructing tissue in vitro, there is a method in which the cells to be cultured are cultured in the lumens of hollow fibers each comprising a permeable membrane. This is a method in which cells are cultured in the sealed lumens of the hollow fibers which are optionally filled with a collagen gel, an agarose gel or the like, whereby 3-dimensional culture of cells or tissue is possible. With this method, it is possible to supply nutrients to the cells and eliminate waste matter discharged from the cells efficiently, by putting the cells into the hollow fiber lumens, followed by perfusing a culture solution around the outside of the hollow fibers. Moreover, due to the cells being enveloped by the hollow fiber membranes, there is also an advantage that the cells can be protected from physical damage caused by stream of the culture solution.

However, with all of these culture methods, it is necessary to use a large perfusion apparatus that assumes an in vitro artificial organ. That is, to perfuse the culture solution around the outside of the hollow fibers, special apparatus including a housing enveloping the hollow fibers and a pump to perfuse the culture solution is required, and hence it has been very difficult to carry out tests for investigating the functions or metabolism of cells.

As a specific example of preparing a medical biomaterial by reconstructing tissue through in vitro culture of cells that have been isolated from a living body, trials have been carried out into preparing an epidermal layer or a corneal layer by culturing epidermal keratinocytes on a collagen gel containing fibroblasts.

Moreover, Japanese Patent Application Laid-open No. 2002-247978 discloses, as a method of manufacturing a hepatocyte organoid for use as an artificial liver, a method in which hepatocytes are injected into the inside or outside of hollow fibers, and then the cells are packed to high density by applying centrifugal force or hydrostatic pressure.

Furthermore, various studies have been carried out into a method in which cells are reconstructed into a 3-dimensional structure by embedding the cells in a collagen gel or filling the cells into a hollow fiber module, and then culture is carried out in this state to prevent loss of functions during culture (e.g. Japanese Patent Application Laid-open No. 2001-128660).

Furthermore, in recent years regenerative medical techniques in which cell transplantation therapy is carried out using cells with regenerative ability that are present in the human body have received attention, and it is expected that it will be possible to regenerate the functions of body tissues and organs that have become dysfunctional using such regenerative medical techniques.

In regenerative medical techniques, it is essential to control cell differentiation, and it is known that failure of the cell differentiation mechanism can cause tumorous diseases and so on. Consequently, in regenerative medical techniques, there are calls for technology for carrying out differentiation control such that a group of undifferentiated cells is efficiently induced to differentiate in a predetermined differentiation direction.

With a conventionally carried out cell culture method such as monolayer culture (2-dimensional culture), it is difficult to efficiently induce a variety of cells to differentiate in a predetermined differentiation direction. For example, it is known that, in the case of carrying out monolayer culture of cartilage cells, even if the culture is carried out under conditions in which differentiation is induced, some of the cells will secrete substances characteristic of cartilage cells and also exhibit a polygonal form, but most of the cells will exhibit a fibroblastic form in which the characteristics of cartilage cells have been lost.

Moreover, it has been reported that if some kind of cartilage inducing agent or factor is brought into contact with centrifuged pellets obtained by applying centrifugal force to human mesenchymal stem cells in a vessel and thus binding the cells together into a 3-dimensional form, then differentiation takes place along a chondrogenesis pathway (see, for example, Published Japanese Translation of PCT Application No. 2000-516802). However, with this method, cell aggregates having homogeneous functions for differentiation cannot be obtained.

Furthermore, as methods of transporting or storing cells, the following two methods are predominantly used.

One is a method in which cells suspended in a culture medium for freezing are frozen in a freezing vial or ampoule, and then transportation or storage is carried out in this state. The other is a method in which cells are cultured in a vessel for cell culture such as a flask to make the cells adhere to or proliferate on the vessel walls, and then the vessel is filled with a culture solution or the like, and the vessel is sealed, and transportation or storage is carried out in this state at room temperature.

With fibroblasts and so on, which have a strong ability to proliferate regardless of whether they are an established cell line or a primary cell culture, the cell functions are not easily lost upon transporting or storing using the above-mentioned methods.

However, functions of the cells are likely to be lost during transportation of the cells in the form of monolayer culture in a flask which is filled with culture solution.

It is an object of the present invention to provide methods according to which cells can be seeded, cultured, stored, transported, and induced to differentiate with the functions of the cells being maintained.

It is another object of the present invention to provide an instrument for seeding, culturing, storing, transporting, and inducing differentiation in cells with the functions of the cells being maintained, and a method of using the instrument.

### BRIEF DESCRIPTION OF THE DRAWINGS

FIG. 1 is a sectional view of a hollow fiber-possessing instrument according to an embodiment of the present invention;
FIG. 2 is a sectional view of a hollow fiber-possessing instrument according to another embodiment of the present invention;
FIG. 3 is a sectional view of a hollow fiber-possessing instrument according to yet another embodiment of the present invention;
FIG. 4 is a graph showing the change in the ammonia metabolizing capability of cell aggregates prepared in hollow fiber lumens using the hollow fiber-possessing instrument of the present invention (Example 1) and cells prepared by monolayer culture (Comparative Example 1);
FIG. 5 is a graph showing the change in the albumin producing ability of cell aggregates prepared in hollow fiber lumens using the hollow fiber-possessing instrument of the present invention (Example 1) and the cells prepared by monolayer culture (Comparative Example 1);
FIG. 6 is a graph showing the amount of albumin production per unit number of cells for cells subjected to a transportation test;
FIG. 7 is a graph showing the amount of ammonia metabolism per unit number of cells for cells subjected to a transportation test;
FIG. 8 is a graph showing an amount of albumin production per unit number of cells;
FIG. 9 is a graph showing an amount of ammonia metabolism per unit number of cells;
FIG. 10 shows toluidine blue stained images of a cartilage cell culture obtained by inducing differentiation using an example of a differentiation inducing method of the present invention;
FIG. 11 shows toluidine blue stained images of a cartilage cell culture obtained by inducing differentiation using a conventional monolayer culture method;
FIG. 12 is a graph showing the amount of neutral fat accumulated in cells obtained in Example 2D and Comparative Example 2D;
FIG. 13 is a drawing showing an example of a state in which a culture insert has been installed in a multi-well plate 7, and a cell aggregate 9 has been formed on a permeable membrane 81 in each well of the culture insert 8;
FIG. 14 is a graph showing the amount of albumin production of cells per unit number of cells;
FIG. 15 is a graph showing the amount of ammonia metabolism of cells per unit number of cells;
FIG. 16 is a graph showing relative values of type II collagen mRNA expression amount per unit number of cells for a culture of aggregates of cartilage cells and a culture obtained by monolayer culture of cartilage cells; and
FIG. 17 is a toluidine blue stained image of a section of a tissue-like body formed in the cartilage cell aggregate culture.

### SUMMARY OF THE INVENTION

In an embodiment of the present invention, the present inventors carried out various studies, and discovered that loss of the functions of the cells can be suppressed by applying centrifugal force or pressure such as hydraulic pressure to cells to form cell aggregates on permeable membranes such as hollow fiber membranes, followed by culturing, storing, or inducing differentiation in the cells in this aggregate state. Moreover, the present inventors discovered that the loss of the functions of the cells during storage can be suppressed yet more effectively by culturing such cell aggregates in a culture medium for cell culture to form tissue bodies, followed by storing these tissue bodies.

In another embodiment of the present invention, the present inventors discovered that in the case of adhering cell aggregates to permeable membranes such as hollow fiber membranes, making the surface of each permeable membrane on which the cell group is not adhered come into contact with a culture solution in a vessel, and culturing the cell aggregates in this state while moving the vessel continuously or intermittently, the culture solution can be made to flow around the vessel easily, and as a result nutrients can easily be provided to the whole of each cell aggregate, and moreover gas exchange can be carried out, and as a result the cells can be cultured easily while maintaining the functions of the cells over a prolonged period; in particular, in the case of forming cell aggregates on permeable membranes such as hollow fiber membranes, or forming cell aggregates in a collagen gel, the cell functions are maintained yet more efficiently.

In another embodiment of the present invention, the present inventors discovered that the whole of the undifferentiated cell group can efficiently be made to differentiate uniformly in a predetermined direction by applying centrifugal force or pressure to undifferentiated cells via vessel walls, particularly permeable membranes such as hollow fiber membranes, to form cell aggregates adhered to the permeable membranes, followed by culturing these aggregates. Moreover, the present inventors discovered that differentiation can be induced to occur in a predetermined direction yet more efficiently by adding components that induce cell differentiation to the aggregate culture environment.

Here, 'differentiate in a predetermined direction' includes the following cases (a) to (c):
(a) as opposed to the case that the undifferentiated cells differentiate in the same direction and yet the rate differs and hence as a result a group of cells having different differentiation functions is formed, the case that the whole cell group can be made to differentiate at the same rate and hence as a result a group of cells having homogeneous differentiated functions is formed;
(b) the case that differentiation of the undifferentiated cells proceeds in the same direction as opposed to in differing directions; and
(c) the case that differentiation proceeds in a normal differentiation direction, with there being no canceration.

The detailed mechanism of the induction of differentiation is not completely clear, but the present inventors conjecture that the frequency of contact between cells increases by filling cells having differentiation potential into hollow fibers or the like to high density, followed by applying centrifugal force or pressure to the cells, and as a result phenomena such as accentuation of inter-cell information transfer and acquisition of cell position information come to occur more easily, and hence a microenvironment in which the cells differentiate normally is created.

In another embodiment of the present invention, the present inventors discovered that aggregates are formed in which the cells are piled up on top of one another in layers and moreover a state of high contact or a high contact frequency is maintained between the cells by applying centrifugal force or pressure when seeding cells into a culture vessel, and that the cells can be cultured while maintaining the functions of the cells at a high level over a prolonged period by culturing the cells in this aggregate state.

In another embodiment of the present invention, the present inventors discovered that aggregates are formed in which a state of high contact or a high contact frequency is maintained between the cells by applying centrifugal force or pressure such as hydraulic pressure to cells, followed by culturing the cells in this aggregate state, consequently the cells can be cultured over a prolonged period while being made to exhibit to a high degree the functions originally possessed by the cells. Moreover, the present inventors discovered that a tissue body is formed relatively rapidly by culturing such cell aggregates in a culture medium for cell culture, and that the expression and maintenance of the cell functions during culture are realized yet more effectively by culturing this tissue body.

The present invention provides the following cell seeding, culture, storage and differentiation inducing methods, cell culture, cell culture instrument;
Item 1: A hollow fiber-possessing instrument, comprising:
   a cell suspension flow tube (1) having one end open;
   a hollow fiber fixing part (3) provided in the other end of the cell suspension flow tube (1); and
   one or a plurality of hollow fibers (4) that each has one end sealed and the other end open, and each passes through the hollow fiber fixing part (3) such that the open end of the hollow fiber (4) communicates with the cell suspension flow tube (1) and liquid does not leak.
Item 4: A preferred embodiment of the instrument of item 1, further comprising a centrifuging vessel (6) inside which the hollow fibers (4) can be disposed, wherein a lid (62) of the centrifuging vessel (6) is supported by the cell suspension flow tube (1).
Item 6: A method of using the hollow fiber-possessing instrument according to item 1 or 4, comprising injecting a cell suspension from the open end of the cell suspension flow tube (1) of the hollow fiber-possessing instrument to accumulate cells in a lumen of each of the hollow fiber (4) or fibers (4), and culturing or storing the cells in a state in which the hollow fibers (4) having the cells accumulated therein are immersed in a cell culture solution or a solution for cell storage, provided that the cells are not human embryonic cells.
Item 8: A method of using the hollow fiber-possessing instrument according to item 4, comprising the steps of: injecting a cell suspension from the open end of the cell suspension flow tube (1) of the hollow fiber-possessing instrument to accumulate cells in a lumen of each of the hollow fibers (4);
   applying a centrifugal force to the cells in the hollow fiber lumens in a state in which the hollow fibers (4) are disposed in the centrifuging vessel (6) to form cell aggregates (9) in the hollow fiber lumens; and
   culturing or storing the cells in a state in which the hollow fibers (4) holding the cell aggregates (9) are immersed in a cell culture solution or a solution for cell storage, provided that the cells are not human embryonic cells.
Item 10: A method for seeding cells in which a drop in cell functions is suppressed, comprising the steps of:
   (a) putting a cell culture solution having one or more types of cells in a culture medium into a lumen or lumens of a porous hollow fiber (4) or fibers (4); and
   (b) applying pressure or centrifugal force to the cells in a vessel to form a cell aggregate in a lumen or lumens of the hollow fiber (4) or fibers (4), which is set in a hollow fiber-possessing instrument described in item 1, provided that the cells are not human embryonic cells.
Item 20: A method for culturing cells in which a drop in cell functions is suppressed, the method further comprising a step of culturing the cell aggregates (9) formed in item 10.
Item 21: A method for culturing cells comprising bringing the hollow fibers (4) having formed therein the cell aggregates (9) obtained using the method of item 10 into contact with a culture solution in a vessel, and culturing cells in this state while moving the vessel continuously or intermittently.
Item 28: A method for storing cells for which a drop in cell functions is suppressed, the method further having a step of storing the cell aggregates formed in claim 10.

### DESCRIPTION OF THE PREFERRED EMBODIMENTS.

Following is a detailed description of the present invention.

### (1) Permeable membranes such as hollow fibers

One embodiment of the seeding method of the present invention is a method for forming aggregates each comprising a plurality of layers of the cells on the permeable membranes, wherein centrifugal force is applied to the cells in a state in which cells have been placed on the permeable membranes of a vessel having permeable membranes therein.

When forming the aggregates on the permeable membranes, components necessary for the survival of the cells can be supplied in efficiently while maintaining the aggregate state by making the cells contact a liquid medium for cell culture or a solution for organ transportation via the permeable membranes. As a result, the cells can be cultured, stored, or induced to differentiate while maintaining the functions possessed by the cells.

There are no particular limitations on the form of the permeable membranes, with examples being a membrane form, a hollow fiber form, a bag form having one or more openings, and so on. It is particularly preferable to use hollow fibers.

In the following description, hollow fibers, which are particularly preferable as permeable membranes, will predominantly be taken as examples, but application is similarly possible with other permeable membranes.

Each hollow fiber is constituted from a membrane having a large number of pores that do not allow the passage of cells but do allow the passage of culture solution components such as water, salts and proteins; the pores generally preferably have a size of approximately 0.1 to 5 µm, particularly preferably approximately 0.2 to 3 µm .

There are no particular limitations on the thickness of the hollow fibers, so long as this thickness is in a range such that a suitable strength and a good permeability to substances are maintained; it is generally preferable to adopt a thickness of approximately 10 to 200 µm, particularly preferably approximately 20 to 100 µm.

There are no particular limitations on the material of the hollow fibers, so long as this material is not cytotoxic, and is not degenerated or decomposed through sterilization, cleaning, or bringing into contact with culture solutions or the like. Examples include cellulose resins, polyolefins such as polyethylene and polypropylene, polysulfones, polyethersulfones, fluororesins, polycarbonates, acrylic resins, and so on. Alternatively, hollow fibers made of a biodegradable resin and/or a biocompatible resin can be used, and in this case, after the aggregates have been formed, transplantation of cells into a living body can be suitably carried out with hollow fibers.

The cell aggregates formed in the hollow fibers are cultured, stored, or induced to differentiate in a state in which the hollow fibers are immersed in a culture solution. There are no particular limitations on the vessel in which the cell aggregates formed in the hollow fibers are cultured, stored, or induced to differentiate; for example, a petri dish, a multi-well plate, or the like can be suitably used.

In the case of a cell aggregate formed in a hollow fiber, if a liquid medium for cell culture, a differentiation-inducing culture medium, or a solution for organ transportation is supplied to the aggregate via the hollow fiber, then components necessary for the survival of the cells can be supplied in efficiently while maintaining the aggregate state. As a result, the cells can be cultured, stored, or induced to differentiate while maintaining the functions possessed by the cells. Moreover, by using a hollow fiber, the cell aggregate is set apart from the liquid culture medium or the like, and hence the cells can be protected from physical shock due to movement or flow of the liquid during storage such as transportation. Moreover, the cells can be stored at a high density.

There are no particular limitations on the size of each hollow fiber, but so that nutrients can be supplied efficiently to the cells inside the hollow fiber, the inside diameter of the hollow fiber is generally preferably approximately 20 to 1000 µm, particularly preferably approximately 50 to 500 µm, more preferably approximately 100 to 300 µm. If the inside diameter is within such a range, then substance exchange can be carried out sufficiently even for cells located in a central part of the hollow fiber, and yet a sufficient number of cells can be put into the hollow fiber for practical purposes. There are no particular limitations on the length of each hollow fiber, but this length can be made to be, for example, approximately 0.5 to 20 cm, in particular approximately 1 to 10cm.

In one preferred embodiment of the present invention, a cell culture module in which a large number of hollow fibers are arranged regularly at minute intervals inside a shell is used, and a cell suspension is injected into the hollow fiber lumens, and then centrifugal force is applied to the cells with the module and all using a centrifuge, whereby an aggregate can be formed in each hollow fiber lumen. Next, the cells can be cultured, stored, or induced to differentiate in a state in which the part inside the shell on the outside of the hollow fibers is filled with a liquid medium for cell culture, a differentiation-inducing culture medium, a solution for organ storage, or the like.

There are no particular limitations on the liquid medium for cell culture used when culturing, storing, or inducing differentiation in the aggregates; for example, a conventional basal culture medium for cell culture such as Dulbecco's modified eagle medium, Williams' E medium, Ham's F-10 medium or F-12 medium, an RPMI-1640 medium, an MCDB 153 medium, or a 199 medium can be used, with conventional growth factors, antioxidants and so on suitable for culture of the cells in question being added thereto as required.

Moreover, as a solution for storage of organs, a conventional solution for organ storage such as a Euro-Collins solution, a Ficoll-Collins solution, a UW solution (Japanese Patent Publication No. H7-68082), or a raffinose solution can be used.

Furthermore, the cells can be cultured, stored, or induced to differentiate in a state in which the aggregates are enveloped by a collagen gel, an agarose gel or the like containing a liquid medium for cell culture, a differentiation-inducing culture medium, or a solution for organ storage. As a result, the cells can be protected yet more effectively from shock during transportation.

The temperature during the culture varies according to the type of the cells, but is generally preferably made to be approximately 36 to 37°C. The culture time period varies according to the usage of the cell culture obtained, but is generally preferably at least approximately 4 hours, particularly preferably approximately 12 hours to 1 week.

By culturing to such an extent, a cell culture can be obtained that exhibits well the functions that were possessed by the cells in vivo.

During storage, the hollow fibers having the aggregates formed therein should, for example, be put into a suitable vessel together with a liquid culture medium or the like. The vessel may have air vents, or may be hermetically sealed.

The temperature during the storage should be selected as appropriate in accordance with the type of liquid culture medium or solution for organ transportation used during the storage. For example, in the case of using one of the liquid media for cell culture given as examples earlier, it is preferable to carry out the storage at room temperature or below. That is, it is generally preferable to carry out the storage at a temperature of approximately 5 to 35°C. Moreover, in the case of using a solution for organ transportation suitable for transportation at low temperature, it is generally preferable to carry out the storage at a temperature of approximately 0 to 10°C.

There are no particular limitations on the use applications of cells that have been cultured or stored according to the present invention, but examples include cell function tests, metabolizing capability tests, tests on the production of a substance using cells, drug screening tests using the above, and so on. Moreover, cells that have been stored can be used as an artificial organ.

Moreover, using a cell culture module as described above, it is also possible to form cell aggregates in the gaps between the shell and the hollow fibers, and then culture, store, or induce differentiation in the cells in a state in which the hollow fiber lumens are filled with a liquid medium for cell culture, a differentiation-inducing culture medium, a solution for organ storage or the like.

Moreover, it is also possible to form cell aggregates in the hollow fiber lumens, and then cut off the hollow fibers, seal both ends of each hollow fiber by compression bonding, ligation or the like, and then culture, store, or induce differentiation in the cells in a state in which the hollow fibers are immersed in a liquid medium for cell culture, a differentiation-inducing culture medium, or a solution for organ storage.

Regarding the permeable membranes, hollow fibers are particularly preferable, but in the case of a form other than hollow fibers, the permeable membranes can be used in a membrane shape as is. Regarding the magnitude of the centrifugal force or pressure, the time for which the centrifugal force or pressure is applied, and the number of cells placed on the permeable membranes, these are the same as described later for the cell seeding method of the present invention. For example, it is possible to use a vessel in which the whole or part of a bottom surface of the vessel is constituted from permeable membranes, a vessel in which the whole or part of side surfaces as well as the bottom surface of the vessel are constituted from permeable membranes, a vessel that has a plurality of wells wherein the whole or part of a bottom surface of each well is constituted from a permeable membrane, a vessel that has a plurality of wells wherein the whole or part of side surfaces as well as the bottom surface of each well are constituted from a permeable membrane, or the like, and form cell groups on the permeable membranes of the vessel.

In these cases, it is possible to form cell aggregates on the permeable membranes of the vessel, and then fit the permeable vessel into a separate culture vessel, and in this state put a cell culture solution into the culture vessel and culture the cells. Moreover, as will be described later, it is also possible to form the aggregates in a state in which the permeable vessel has been fitted into the culture vessel. Moreover, it is also possible to take the permeable membranes on which the aggregates have been formed out from the vessel, and then transfer the permeable membranes complete with the aggregates into a separately prepared culture solution and carry out culture.

In the case of using one of the above vessels, it is possible to form aggregates by applying pressure to the cells which have been placed on the permeable membranes, and then fit the vessel into a separate culture vessel, pour in a culture solution, and carry out culture as is.

Furthermore, the permeable vessel may be such that a permeable membrane is provided horizontally or approximately horizontally in a central position in the depth direction of the vessel. In this case, a cell suspension is placed on the permeable membrane, and centrifugal force or pressure is applied, whereby liquid passes through the permeable membrane and collects in the vessel. After an aggregate has thus been formed, it is then possible to put a cell culture solution into the vessel and culture as is.

The vessel in which the cells are seeded may also be one having a plurality of wells each having a permeable membrane. In the case of using such a vessel having a plurality of wells, by placing cells on the permeable membrane of each of the plurality of wells and then applying centrifugal force to the cells in this state, a plurality of cell aggregates can be formed at once. Forming a plurality of aggregates on a plurality of permeable membranes in this way is convenient in the case of culturing a plurality of types of cells at once.

In the cell culturing step, the permeable vessel on which the cell aggregates have been formed is fitted into a culture vessel having a size such that the permeable vessel can be fitted therein, and in this state a cell culture solution is filled into the culture vessel and the cells are cultured.

Moreover, it is also possible to fit the permeable vessel into such a culture vessel in advance, and in this state put the cells into the permeable vessel and apply the centrifugal force to the cells. In this case, after the cell aggregates have been formed on the permeable membranes, it is possible to fill the culture solution into the culture vessel and carry out culture as is without moving the permeable membranes on which the aggregates have been formed, which is convenient.

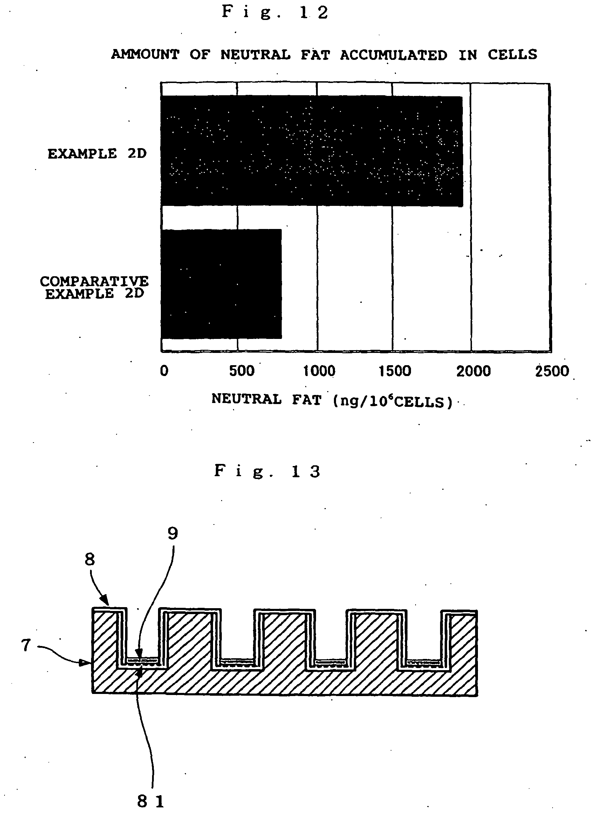

Specifically, as shown in Fig. 13, a permeable vessel (a so-called culture insert 8) that has a plurality of wells wherein a bottom surface of each well is constituted from a permeable membrane is installed in a culture vessel (a so-called multi-well plate 7) having a plurality of wells of a size such that the wells of the permeable vessel can be fitted therein, such that the wells engage with one another; a cell suspension is then put into each of the wells of the culture insert 8, and the multi-well plate 7 having the culture insert fitted therein is centrifuged using a centrifuge so that a centrifugal force is applied to the cells, whereby cell aggregates can be formed on the permeable membranes 81 of the culture insert. An amount suitable for culture of a liquid medium for cell culture is then put into each well of the multi-well plate 7, and the cells are cultured in that state.

A cross section of an example of a state in which a cell aggregate 9 has been formed on each permeable membrane 81 in a state in which the culture insert 8 has been fitted into the multi-well plate 7 is shown in FIG. 13. In this example, the culture insert 8 in which bottom surfaces are constituted from the permeable membranes 81 is installed in the multi-well plate 7 such that the wells fit into one another. Cell aggregates 9 are formed on the permeable membranes 81.

Regarding culture inserts comprising permeable membranes having similar properties, ones having a plurality of wells, for example 6 wells, 12 wells, 24 wells and so on, are commercially available under trade names such as Membrane Culture Insert ( Iwaki Glass).

### (2) Methods of seeding, culturing, storing, and inducing differentiation in cells

A cell seeding method of the present invention is a method in which cells are put into vessels such as hollow fibers, and pressure or centrifugal force is applied to the cells in this state, thus forming aggregates each comprising a plurality of layers of the cells on the inner walls of the vessels. Through this method, the cells can be seeded while suppressing a drop in cell functions. The seeded cells may be differentiated cells, or may be undifferentiated cells.

In general, 'seeding' of cells refers to sowing the cells on inner surfaces (culture surfaces) of vessels (culture vessels); in the present invention, the cells are sown on inner surfaces of vessels (particularly permeable membranes such as a hollow fibers) in the form of dense bodies of cells, preferably aggregates.

In the present invention, 'cell aggregate' refers to a collection of cells assembled together at a density of at least approximately 10⁵ cells/ml, although this varies according to the type and size of the cells. There are no particular limitations on the upper limit of the cell density in the cell groups, but this is generally up to approximately 10¹⁰ cells/ml. The cell groups may comprise one type of cells, or may comprise a plurality of types of cells. A method of putting cell aggregates into a gel such as a collagen gel is known as described, for example, in 'Tissue Culture Techniques' (Japanese Tissue Culture Association, 3rd edition 2nd printing, p271-273). In addition, cell aggregates can be formed, for example, in hollow fibers by injecting into the hollow fibers a cell dispersion in which cells have been dispersed at a high density of, for example, approximately 10⁷ to 10⁹ cells/ml.

Cell aggregates can also be formed by putting cells into the lumens of hollow fibers each comprising a permeable membrane, and in this state applying pressure to the cells by putting the hollow fiber lumens into a positive pressure state or putting the outside of the hollow fibers into a negative pressure state using an injection syringe or the like. The size of the pressure is as described later.

Specifically, for example, a cell culture module in which a large number of hollow fibers are arranged regularly at minute intervals inside a shell is used, and when injecting a cell suspension into the hollow fiber lumens, pressure is applied to the cells using an injection syringe, whereby aggregates can be formed in the hollow fiber lumens. Next, a liquid medium for cell culture or the like is perfused around a part inside the shell on the outside of the hollow fibers, whereby the cells can be cultured.

After forming the cell aggregates on the inner surfaces of the hollow fibers, it is possible to add a cell culture solution and culture as is. Alternatively, supernatant may be removed and cell culture solution further put into the vessel, and then the cells cultured in this state.

'Cell aggregate' refers to a cell group in a state in which the cells are adhered together to a high degree or with a high frequency to the extent that this would not be attainable when dispersed (differentiated or undifferentiated) cells aggregate spontaneously. In each aggregate in the present invention, the cells do not form a single layer, but rather are piled up on top of one another regularly or irregularly, preferably forming a plurality of cell layers. The cells may also be maintained at a high density by being contained in a gel such as a collagen gel or an agarose gel. Furthermore, the cell aggregates may be contained in a gel such as a collagen gel or an agarose gel. In the case of using a gel, the cells may be embedded in the gel, or may be partially exposed from the gel.

The culture can be carried out with the cell functions exhibited/maintained at a yet higher level over a yet longer period by culturing cell groups in a state of cell aggregates or contained in a gel. When putting the cells into vessels comprising permeable membranes or the like, as will be described later, it is preferable to adjust the number of cells put into the vessels such that upon applying pressure aggregates can be obtained in which the cells are piled on top of one another in approximately 2 to 200 layers, preferably approximately 2 to 100 layers (e.g. approximately 5 to 100 layers or approximately 2 to 20 layers), particularly preferably approximately 5 to 10 layers. Note that in the case of hollow fiber membranes, the cells are piled up on top of one another in both the radial direction and the length direction, and hence the smaller the diameter the more the cells are piled up on top of one another in the length direction. In the case that there are too many layers in each aggregate, there will be a lack of nutrients and a lack of gas exchange at cells in the central layers of the aggregate, whereas in the case that there are too few layers, the number of cells will be low and hence it will be hard to form an aggregate in which the cell functions are exhibited sufficiently. If the number of layers is in the range of the present invention, then such problems will not occur.

The number of layers of cells in an aggregate can be checked, for example, by cutting out the vessel wall or permeable membrane (e.g. hollow fiber) on which the layered aggregate is placed, carrying out formalin fixation, and then preparing a paraffin embedded section and observing with a microscope.

A specific preferable example of the cell culture method or differentiation inducing method of the present invention is a method in which hollow fibers each having adhered thereon a cell aggregate comprising one or a plurality of types of differentiated or undifferentiated cells are each made to have the surface thereof on which the cell group is not adhered come into contact with a culture solution in a vessel, and in this state the cells are cultured or induced to differentiate while moving the vessel continuously or intermittently. In this culture method or differentiation inducing method, the cells are cultured or induced to differentiate in the state of cell aggregates in which the cells have been reconstructed 3-dimensionally in hollow fibers, and hence the cells can be cultured or induced to differentiate while maintaining the cell functions over a prolonged period. Moreover, to efficiently and reliably supply nutrients and oxygen as far as the cells on the inside of each cell aggregate and remove waste matter from the cells on the inside of each cell aggregate, it is effective to make the culture solution that contacts the cell groups via the hollow fibers flow around; with the method of the present invention, the culture solution can be made to flow around easily by moving the vessel.

A preferable embodiment of the cell culture or differentiation inducing method of the present invention is a method comprising the steps of:
applying centrifugal force or pressure to the cells in a state in which one or a plurality of types of differentiated or undifferentiated cells have been put into the lumens of hollow fibers each comprising a permeable membrane to form cell aggregates; and
culturing or storing the differentiated cells by culturing the cell aggregates; or
inducing differentiation in the undifferentiated cells. When forming the cell aggregates in the hollow fibers using this method, for example a cell suspension or the like can be injected into a plurality of hollow fibers at once using an injection syringe or the like via an adapter.

Another preferable cell culture or differentiation inducing method of the present invention is a method comprising the steps of:
applying centrifugal force or pressure to the cells in a state in which one or a plurality of types of differentiated or undifferentiated cells have been put into gaps between the shell and the hollow fibers of a structure comprising a bundle of hollow fibers each comprising a permeable membrane and a shell enveloping the bundle of hollow fibers to form cell aggregates; and
culturing or storing the differentiated cells by culturing the cell aggregates; or
inducing differentiation in the undifferentiated cells. When forming the cell aggregates between the hollow fibers using this method, for example a cell culture module in which a large number of hollow fibers are arranged regularly at minute intervals like blood capillaries inside a cylindrical shell as described, for example, in Japanese Patent Application Laid-open No. 2001-128660 can be used, and a cell suspension or the like can be injected in from an injection port of this cell culture module.

A cell storage method of the present invention is a method in which centrifugal force or pressure is applied to cells to form aggregates, and then the cells are stored in the aggregate state.

'Storage' in the present invention includes storage while transporting. That is, 'storage' includes not only stationary storage, but also transportation in which the cells are moved by truck, train, aircraft, boat, human labor or the like. Stationary storage includes storage in a workplace that handles cells, storage in a store or the like near to such a workplace, and storage in a store or the like at a transportation destination.

The cell aggregates can be formed by suspending the cells (differentiated or undifferentiated) in a suitable liquid culture medium or buffer in a vessel, and in this state using an ordinary centrifuge to apply centrifugal force within a range such that the cells are not damaged in accordance with the type of cells, thus forming cell aggregates on inner surfaces of the vessel. The centrifugal force varies according to the type of cells, but is preferably made to be approximately 2 to 2,000xG, particularly preferably approximately 4 to 500xG. For example, in the case of primary rat hepatocytes, it is generally preferable to apply centrifugal force of 1500xG or less, particularly preferably 400×G or less. Moreover, in the case of primary rat hepatocytes, there are no particular limitations on the lower limit of the centrifugal force so long as the cell aggregates can be formed, but this lower limit is generally approximately 5×G. When forming layered aggregates on the inner surfaces of a culture vessel or on permeable membranes as described later, if the centrifugal force is too high, then the original form and functions of the cells will be lost. Conversely, if the centrifugal force is too low, then contact between the cells will be weak, and cell aggregates for which the cell functions can be exhibited well will not be formed. If the centrifugal force is in the range of the present invention then such problems will not occur. The centrifuging time should be set as appropriate within a range such that the cell aggregates can be formed.

Aggregates are formed on the inner walls of the vessel by applying the centrifugal force, but depending on the type of the centrifuge, aggregates may be formed on a bottom surface of the vessel, or may be formed on a bottom surface and side surfaces of the vessel.

When forming aggregates of undifferentiated cells, the aggregates can be formed in a state with auxiliary cells that are able to control the differentiation of the undifferentiated cells mixed in. It is thought that such auxiliary cells are able to control the differentiation of the undifferentiated cells through signal transduction by intercellular contact, humoral factors secreted by the auxiliary cells, and so on.

The cell aggregates can also be formed by suspending the cells (differentiated or undifferentiated) in a suitable liquid culture medium or buffer, and in this state applying pressure (direct or hydraulic pressure) within a range such that the cells are not damaged in accordance with the type of cells. For example, in the case of forming cell aggregates on permeable membranes, the cells are accumulated on the permeable membranes, and in this state pressure (direct or hydraulic pressure) is applied to the cells by putting the side on which the cells have been accumulated into a positive pressure state or putting the opposite side into a negative pressure state, whereby the cell aggregates can be formed. The pressure varies according to the type of cells, but is generally approximately 0.05 to 50 kg/cm², preferably approximately 1 to 50 kg/cm², more preferably approximately 5 to 35 kg/cm², particularly preferably approximately 10 to 20 kg/cm². The pressure can be applied using, for example, an injection syringe, an aspirator (water jet pump), an electric pump or the like. So long as the aggregates can be formed, the pressure may be either a negative pressure or a positive pressure.

For example, in the case of forming cell aggregates in hollow fibers, a suspension obtained by suspending the cells in a suitable liquid culture medium or buffer is injected into the hollow fibers, and pressure is applied using an injection syringe or the like within a range such that the cells are not damaged in accordance with the type of cells, whereby the cell aggregates can be formed. The pressure varies according to the type of cells, but is preferably approximately 1 to 50 kg/cm², particularly preferably approximately 5 to 35 kg/cm², more preferably approximately 10 to 20 kg/cm². 'Applying pressure' includes applying a positive pressure or a negative pressure to the cells using an injection syringe, an aspirator (water jet pump), an electric pump or the like.

Moreover, in the case, for example, of forming cell aggregates on the permeable membranes of a vessel having permeable membranes, the cell aggregates can be formed by placing a cell suspension on the permeable membranes and then applying centrifugal force in this state. The size of the centrifugal force and the centrifuging time are as described earlier.

A cell aggregate can also be formed by putting a cell suspension into a vessel, not via a permeable membrane or hollow fiber, and applying centrifugal force to the cells in this state. The centrifugal force and the centrifuging time are as described earlier. Pressure is also as described earlier.

Examples of the vessel used in the centrifuging include a general-purpose centrifuging tube, the wells of a vessel having one or a plurality of wells, and so on.

The vessel in which the cells are seeded may be one having a plurality of wells. In the case of using a vessel having a plurality of wells, by putting the cells into the plurality of wells and then applying centrifugal force to the cells in this state, a plurality of cell aggregates can be formed at once. Forming a plurality of aggregates in a plurality of wells in this way is convenient in the case of culturing a plurality of types of cells at once.

After the cell aggregates have been formed on the inner walls of the vessel or the inner walls of the wells of the vessel, culture canbe carried out as is or after adding a cell culture solution. Alternatively, supernatant may be removed, and cell culture solution further put into the vessel or the wells, and then the cells cultured in this state. As a result, the culture can be carried out while exhibiting or maintaining the functions originally possessed by the cells well.

### (3) Cells

There are no particular limitations on the cells used in the cell seeding, culture, differentiation inducing, and storage methods, and the cell culture, of the present invention, but adhesive human animal cells are preferable. There are no particular limitations on the origin of the cells, with it being possible to use cells originating from any animal, for example a human, a mouse, a rat and so on. Moreover, as adhesive animal cells, either primary cultured cells or an established cell line can be used. The methods of the present invention are particularly suitable for the storage of primary cultured cells, for which maintaining the functions of the cells is difficult. The primary cultured cells may originate from any tissue out of cartilage, bone, skin, nerve tissue, oral tissue, alimentary canal, liver, pancreas, kidney, glandular tissue, adrenal, heart, muscle, tendon, fat tissue, connective tissue, reproductive organ, eyeball, blood vessel, bone marrow and blood. Specifically, for example, cartilage cells, osteoblasts, epidermal keratinocytes, melanocytes, nerve cells, neural stem cells, gliacytes, hepatocytes, intestinal epithelial cells, pancreatic beta cells, pancreatic exocrine cells, renal glomerular endothelial cells, tubular epithelial cells, mammary gland cells, thyroid gland cells, salivary gland cells, adrenocortical cells, adrenomedullary cells, myocardial cells, skeletal muscle cells, smooth muscle cells, fat cells, fat precursor cells, crystalline lens cells, corneal cells, vascular endothelial cells, bone marrow stromal cells, lymphocytes and so on can be used. One type of cells may be used alone, or a plurality of types may be used in combination. In particular, human hepatocytes, which rapidly lose differentiated functions upon monolayer culture, are suitable as cells to be used with the methods of the present invention. As the cells, a single type of cells originating from simple tissue can be used, but it is also possible to use a plurality of types of cells having different origins. In the case of an established cell line, there are no particular limitations thereon, with it being possible to use a cell line such as CHO cells, Vero cells, MRC-5 cells, BHK cells, or HeLa cells, or a cell line obtained by introducing a foreign gene into such cells, and so on.

### (4) Undifferentiated cells

In the method of the present invention, 'cell differentiation' refers to the phenomenon of undifferentiated cells changing into cells having specific functions. Differentiation includes both the process in which finally functioning cells are produced from undifferentiated cells that have not differentiated at all or have an extremely low level of differentiation (e.g. stem cells), and the process in which already differentiated cells having specific functions acquire new functions in accordance with an external stimulus.

Undifferentiated cells that can be used in the method of the present invention are cells that have not reached a final differentiated state. There are no particular limitations on the undifferentiated cells, but examples are cells such as embryonic stem cells, ectodermal stem cells, mesodermal stem cells, endodermal stem cells, mesenchymal stem cells, hematopoietic stem cells, neural stem cells, hepatic stem cells, muscle stem cells, pancreatic stem cells, cutaneous stem cells, retinal stem cells, follicular stem cells, bone precursor cells, fat precursor cells, cartilage cells, hair matrix cells, epithelial cells, vascular endothelial cells, and smooth muscle cells, and cells in the differentiation lineage from these cells.

Moreover, from the standpoint of possessing differentiation potential, cancer cells are also included under undifferentiated cells. Such cancer cells include, for example, MC3T3-E1 cells (a cell line that differentiates into osteoblasts to produce bone), and MC3T3-G2/PA6 cells (which differentiate into fat cells). In this way, cells that are part way through differentiation and have not reached a final differentiated state, i.e. cells that can acquire new functions in accordance with an external stimulus, are also included under undifferentiated cells.

Moreover, there are no particular limitations on the origin of the cells, although the cells preferably originate from an animal and human, particularly a mammal. There are no particular limitations on the type of mammal, with it being possible to use undifferentiated cells originating from any animal, for example a human, a mouse, a rat and so on, as the undifferentiated cells in the present invention. It is particularly preferable to use cells originating from a human.

One type of undifferentiated cells can be used alone, or a plurality of types can be used in combination.

### (5) Method of culturing or inducing differentiation in cells while moving vessel

The culture of cell aggregates formed in hollow fibers can be carried out while moving the vessel containing the hollow fibers continuously or intermittently, thus making the culture solution flow within the culture vessel. The culture vessel can be moved in a horizontal direction by being rotated or turned in a circular or polygonal shape, or can be moved back and forth in a horizontal direction. Rotation includes not only the case of rotating in a single direction, but also the case of rotating alternately in opposite directions and so on. Back and forth movement includes not only the case of moving back and forth in a single direction, but also the case of moving back and forth at angles and so on. Moreover, back and forth movement includes not only the case of back and forth movement in which the original position is completely returned to, but also the case of incomplete back and forth movement. Further, seesaw movement, combinations of seesaw movement and above-mentioned movements are also included.

During the culture or differentiation induction, the whole of the hollow fibers having the cell aggregates formed therein may be in contact with or immersed in the culture solution, or the hollow fibers may be partially exposed from the culture solution.

' There are no particular limitations on the equipment for moving the culture vessel, but a commercially available shaker can be suitably used. In the case of using a culture vessel in which the part or parts into which the culture solution is put is/are circular or elliptical such as a petri dish or a multi-well plate, a shaker that carries out turning motion is preferable.

In the case of turning motion, the speed at which the culture vessel is moved is generally preferably made to be approximately 1 to 200rpm, particularly preferably approximately 10 to 100rpm. If the speed is within such a range, then nutrients can be supplied to the cells efficiently, but the cells will not be damaged. Moreover, in the case of moving the culture vessel back and forth, the speed of oscillation varies according to the volume and shape of the culture vessel, the amount of culture solution in the vessel and so on, but is generally preferably made to be approximately 1 to 200 cycles/min, particularly preferably approximately 10 to 100 cycles/min. Moreover, the amplitude of oscillation varies according to the speed of oscillation, the volume and shape of the culture vessel, the amount of culture solution in the vessel and so on, but in the case, for example, of a petri dish having a diameter of approximately 3cm to 10cm, at a speed of approximately 10 to 50 cycles/min, an amplitude of approximately 1 to 3cm is appropriate.

There are no particular limitations on the culture solution; for example, a conventional basal culture medium for cell culture such as Dulbecco's modified eagle medium, Williams' E medium, Ham's F-10 medium or F-12 medium, an RPMI-1640 medium, an MCDB 153 medium, or a 199 medium can be used, with conventional growth factors, antioxidants and so on suitable for culture of the cells in question being added thereto as required.

The temperature during the culture or differentiation induction varies according to the type of the cells, but is generally preferably made to be approximately 36 to 37°C. The time for which the culture or differentiation induction is carried out varies according to the usage of the cell culture obtained, but is generally preferably made to be at least approximately 12 hours, particularly preferably approximately 24 to 96 hours.

### (6) Inducing differentiation of cell aggregates

### <In vitro>

When culturing cell aggregates, the culture can be carried out in a culture medium for cell culture. There are no particular limitations on the culture medium for cell culture, with it being possible to use, for example, a culture medium that has been known from hitherto as a basal culture medium for cell culture, for example Dulbecco's modified eagle medium, Williams' E medium, Ham's F-10 medium or F-12 medium, an RPMI-1640 medium, an MCDB 153 medium, a 199 medium, or the like. By adjusting the components of the culture medium as appropriate, undifferentiated cells can be made to differentiate into desired cells.

Moreover, differentiation-inducing components can be added to the culture medium, whereby it becomes possible to induce the undifferentiated cells to differentiate into the desired differentiated cells more efficiently. As the differentiation-inducing components, conventional differentiation-inducing components can be added in accordance with the type of the undifferentiated cells and the type of the desired differentiated cells.

Examples of such components include, for example, interleukin (IL), stem cell growth factors (SCFs), erythropoietin (EPO), interferon (IFN), thrombopoietin (TPO), tumor necrosis factors (TNFs), colony stimulating factor (CSFs), and so on, which are differentiation-inducing components for hematopoietic stem cells. Moreover, as factors for inducing differentiation from mesenchymal stem cells to bone, examples include dexamethasone, β-glycerol phosphate, ascorbic acid, and so on. As a factor for inducing differentiation from mesenchymal stem cells to cartilage cells, TGF-β (transforming growth factor-β) is preferable. Moreover, as components for inducing differentiation from mesenchymal stem cells to fat cells, examples include dexamethasone, 1-methyl-3-isobutylxanthine, which is a phosphodiesterase inhibitor, insulin, indomethacin, and so on.

The culture temperature should be made to be a suitable temperature in accordance with the type of the undifferentiated cells and the type of the desired differentiated cells. The culture time varies according to the type of the undifferentiated cells and the type of the desired differentiated cells, but is preferably made to be approximately 24 hours to 6 months.

For example, in the case of inducing mesenchymal stem cells to differentiate into cartilage cells, it is appropriate to use a liquid culture medium containing Dulbecco's modified eagle medium as a culture medium, add suitable amounts of TGF-β, insulin, transferrin and dexamethasone or the like as differentiation-inducing components, and culture for approximately 2 to 4 weeks at approximately 37°C.

Moreover, in the case of inducing mesenchymal stem cells to differentiate into fat precursor cells, it is appropriate to use a liquid culture medium containing Dulbecco's modified eagle medium as a culture medium, add suitable amounts of dexamethasone, insulin and indomethacin or the like as differentiation-inducing components, and culture. Furthermore, in the case of inducing mesenchymal stem cells to differentiate into skeletal muscle cells, it is appropriate to use a liquid culture medium containing Dulbecco's modified eagle medium as a culture medium, add a suitable amount of 5-azacytidine or the like as a differentiation-inducing component, and culture.

Moreover, in the case that undifferentiated cells differentiate, passing through cells A and then becoming cells B, by changing over to a culture medium not containing differentiation-inducing components after differentiation has taken place as far as the cells A, it is possible to make the whole of the cell group be in the state of the cells A.

In the case that cell aggregates have been formed in hollow fibers, culture may be carried out by immersing the hollow fibers themselves in a liquid culture medium. Moreover, in the case that aggregates have been formed in the hollow fibers of a structure comprising a bundle of hollow fibers in a shell, culture can be carried out by perfusing a liquid medium for cell culture into the gaps between the hollow fibers in the shell. Furthermore, in the case that the aggregates have been formed between the hollow fibers of such a structure, the culture may be carried out by perfusing a liquid medium for cell culture into the hollow fibers.

In the case of using hollow fibers, components necessary for the differentiation, other nutrients, oxygen and so on can be supplied efficiently to the cells, and waste matter can be discharged efficiently, via the hollow fibers, which each comprise a permeable membrane.

### <In situ / in vivo>

Moreover, aggregates of undifferentiated cells can also be induced to differentiate by transplanting the cell aggregates into a living animal and then culturing in this state. In this case, it is appropriate, for example, to transplant hollow fibers having the cell aggregates formed therein into the tunica of tissue such as the liver or the abdominal cavity of an animal such as a mouse or a rat, and then rear this animal for at least approximately 24 hours. As a result, the undifferentiated cell aggregates can be induced to differentiate into differentiated cells.

By transplanting the cell aggregates into the vicinity of specific tissue, the cell aggregates can be induced to differentiate into cells of this specific . tissue through differentiation factors discharged by tissue cells in the vicinity of the aggregates, or signals received from cells contacting the aggregates. For example, in the case of transplanting aggregates of undifferentiated nerve cells into the spinal cord, nerve fibers are formed.

In addition, a differentiation environment that could not be reproduced in vitro can be obtained by transplanting cell aggregates into a living body. In this case, the aggregates are not necessarily transplanted into the vicinity of tissue the same as the tissue to be obtained by differentiation. For example, undifferentiated cells having the potential to differentiate into cartilage will differentiate into cartilage upon being transplanted into the vicinity of muscle tissue.

### <Coculture>

In the case of adopting any of the culture methods, the cell aggregates can be cultured together with other cells in the same culture system. For example, in the case of inducing cells A to differentiate, by culturing, in the same culture vessel, aggregates of the cells A, and cells B that discharge a factor that induces the differentiation of the cells A, the differentiation of the cells A can be promoted. In this case, the cells B may, for example, be filled into the hollow fibers, or may be cultured in the vessel not via a permeable membrane. In the case of culturing the cells B not via a permeable membrane, for example the cells B may be subjected to monolayer culture on a bottom surface of the vessel. The cells B may also be formed into cell aggregates.

### (7) Cell culture or tissue body

A cell culture or tissue body of the present invention can be obtained by culturing cells seeded using the cell seeding method of the present invention.

There are no particular limitations on the liquid medium for cell culture used when culturing the aggregates; for example, a conventional basal culture medium for cell culture such as Dulbecco's modified eagle medium, Williams' E medium, Ham's F-10 medium or F-12 medium, an RPMI-1640 medium, an MCDB 153 medium, or a 199 medium can be used, with conventional growth factors, antioxidants and so on suitable for culture of the cells in question being added as necessary.

Furthermore, the cells can be cultured in a state in which the aggregates have been enveloped by a collagen gel, an agarose gel or the like containing a liquid medium for cell culture. As a result, the cells can be protected from physical damage during medium exchange and so on.

The temperature during the culture varies according to the type of the cells, but is generally preferably made to be approximately 36 to 37°C. The culture time varies according to the usage of the cell culture obtained, but is generally preferably made to be at least approximately 12 hours, particularly preferably approximately 24 to 96 hours.

By culturing to such an extent, a cell culture can be obtained that exhibits well the functions that were possessed by the cells in vivo. The cell culture obtained can be used, for example, in cell function tests, metabolizing capability tests, tests on the production of a substance using cells, drug screening tests using the above, and so on. Moreover, the cell culture can be used as an artificial organ in a state contained in a bag comprising a permeable membrane.

In a method of the present invention, aggregates are formed, the aggregates are then cultured in a liquid medium for cell culture to form tissue bodies, and storage is carried out in the tissue body state, whereby storage can be carried out while maintaining the functions of the cells more effectively.

As the culture medium when forming the tissue bodies, for example a liquid medium for cell culture as described earlier can be used. The culture temperature and the culture atmosphere should be selected as appropriate in accordance with the cells. Moreover, there are no particular limitations on the culture time so long as tissue bodies are formed, but it is generally preferable to make this culture time be at least approximately 12 hours. If the culture time is too long then the cell functions may deteriorate, and hence the upper limit of the culture time is generally approximately 30 days.

The frequency of contact between cells is high in the cell aggregates, and hence the formation of tissue bodies can be carried out easily. For example, in the case that the aggregates are formed in hollow fiber lumens, oxygen and nutrients (the liquid culture medium) are supplied in from outside the hollow fibers, and metabolic waste material is discharged to the outside of the hollow fibers, whereby the cells form a cylindrical tissue body in each hollow fiber lumen. The tissue bodies may be stored as is in the hollow fibers as described earlier.

Moreover, in the case that the aggregates are formed on permeable membranes, it is possible, for example, to form tissue bodies in the wells of a multi-well plate, and then carry out storage by filling a liquid culture medium or the like into the wells, or store the cells in a state in which the tissue bodies are enveloped by a gel containing a liquid culture medium or the like.

### (8) Medical biomaterial

A cell culture or tissue body obtained in this way can be used, for example, as cells for a medical biomaterial. Such a medical biomaterial can be suitably used as a material to replace tissue, an organ or the like that has been lost by an animal such as a human. Depending on the type of the cells cultured, examples of the medical biomaterial include artificial organs such as an artificial liver, an artificial pancreas, an artificial spleen, an artificial kidney or an artificial heart, an artificial alimentary canal, an artificial blood vessel, artificial skin, artificial nerves, artificial bone, artificial cartilage, an artificial inner ear, an artificial lens, an artificial cornea, artificial fat, and so on, or part thereof.

For example, in the case of cell aggregates that have been cultured in the hollow fibers of a cell culture module having a large number of hollow fibers therein, or cell aggregates that have been cultured between the hollow fibers of such a cell culture module and the shell of the module, the module filled with the cell aggregates can be used as an artificial organ such as an artificial pancreas as is. Moreover, it is possible to take the cell culture out from the module, and then use the cell culture as a medical biomaterial for transplantation or the like.

Moreover, in the case of obtaining hepatocyte groups by culturing aggregates of hepatic stem cells in the hollow fibers of a cell culture module having a large number of hollow fibers therein, the module filled with the hepatocyte groups can be used as an artificial liver as is. The module can be used as an in vitro artificial liver by, for example, incorporating the module into an extracorporeal blood circulation system.

Moreover, in the case, for example, of cartilage cell aggregates that have been cultured in hollow fibers, it is possible to take out the formed cell aggregates, and transplant a collection of the cell aggregates either as is or cut into a desired size or shape into a living body at a required site and make the cell aggregates take.

### (9) Transplantation method

A transplantation method of the present invention is a method in which a cell culture or tissue body of the present invention as described above is transplanted into a living human or animal. For example, it is possible to fill undifferentiated cells into hollow fibers each comprising a permeable membrane using centrifugal force to form aggregates, and then transplant the hollow fibers filled with the cell aggregates into a living human or animal. For example, by transplanting fat precursor cell aggregates that have been formed in hollow fibers into an abdominal cavity complete with the hollow fibers, the fat precursor cells can be made to differentiate into fat cells efficiently. Moreover, by filling neural stem cells into hollow fibers each comprising a biodegradable permeable membrane to form aggregates, and then transplanting the neural stem cell aggregates into the spinal cord complete with the hollow fibers, nerve fibers can be expected to be formed. It is thought that this method will become an effective method of treating spinal cord injuries.

### (10) Hollow fiber-possessing instrument of the present invention

A hollow fiber-possessing instrument of the present invention comprises a cell suspension flow tube having one end open, a hollow fiber fixing part provided in the other end of the cell suspension flow tube, and one or a plurality of hollow fibers that each has one end sealed and the other end open, and each passes through the hollow fiber fixing part such that the open end of the hollow fiber communicates with the cell suspension flow tube and liquid does not leak.

### Hollow fibers

As the hollow fibers, hollow fibers as described earlier can be used; the average pore size of the hollow fibers, the inside diameter, the thickness of the semipermeable membrane constituting each hollow fiber, the length of the hollow fibers, and the material of the hollow fibers are as described earlier.

Moreover, there are no particular limitations on the number of hollow fibers, but it is preferable to use approximately 1 to 1,000.

Each hollow fiber has one end open and the other end sealed. There are no particular limitations on the sealing method; for example, the sealing can be carried out using a resin like the resin constituting the hollow fiber fixing part, described later, or the sealing can be carried out using ligation.

### Cell suspension flow tube

The cell suspension flow tube (hereinafter referred to as the 'flow tube') is a tube for injecting a cell suspension into the hollow fiber lumens. The flow tube has a hollow fiber fixing part formed at one end thereof, and the other end is open, thus forming a cell suspension injection port. The inside diameter of the flow tube should be determined in accordance with the thickness of the bundle of hollow fibers, but is generally made to be approximately 1 to 30 mm. Moreover, the length of the flow tube is preferably approximately 1 to 60 mm, particularly preferably approximately 2 to 40 mm. If the flow tube is too short, then it will be difficult to inject cells into the hollow fiber lumens uniformly, whereas if the flow tube is too long, then the number of cells that remain in the flow tube after the cells have been injected into the hollow fiber lumens will be high, and these cells remaining in the flow tube will be wasted. If the length of the flow tube is in the range of the present invention, then such problems will not occur.

The flow tube may be flexible, or may be hard and non-flexible.

The shape of the opening part of the flow tube is preferably such that this opening part can be coupled by external fitting or internal fitting to a discharge port of an instrument for injecting in the cell suspension (e.g. an injection syringe, a micropipette chip, an installment injection tube, or the like). In the case that the opening part has a shape such that coupling to the discharge port of a cell suspension injecting instrument cannot be carried out, an adapter for coupling the opening part of the flow tube and the discharge port of a cell suspension injecting instrument together can be provided on the opening part of the flow tube. The adapter may be fixed, or detachably coupled, to the open end of the flow tube. It is particularly preferable for the adapter to be detachably coupled to the opening part of the flow tube; in this case, when attaching a centrifuging vessel after the cells have been injected in and centrifuging using a centrifuge as will be described later, the adapter can be taken off, and hence handling becomes easier during the centrifuging.

Moreover, the flow tube can be made to comprise two tubes (an inflow side flow tube and an outflow side flow tube) that are detachably coupled together. In this case, there is an advantage that the outflow side flow tube, to which the hollow fibers are fixed, and the inflow side flow tube can be sterilized separately. The inflow side flow tube and the outflow side flow tube may be such that they can be screwed together, or may be such that they are merely fitted together. In either case, the coupling should be carried out such that the cell suspension will not leak out.

There are no particular limitations on the material from which the flow tube (including a coupling member for coupling together the inflow side flow tube and the outflow side flow tube) and the adapter are constituted, so long as it is a material that is not cytotoxic, and is not degenerated or decomposed through contact with sterilizing, cleaning, or culture solutions or the like. For example, a polyolefin resin, a polyester resin, a polystyrene resin, a polycarbonate resin, a polyamide resin or the like can be used.

Moreover, the hollow fiber-possessing instrument of the present invention may have a cap that covers the open end of the flow tube. By covering the opening part of the flow tube with the cap after the cell suspension has been injected in from the flow tube, microbial contamination of the cells and so on can be prevented.

### Hollow fiber fixing part

The hollow fiber fixing part is a part for fixing the hollow fibers to the flow tube in a state in which the hollow fibers communicate with the flow tube. The hollow fiber fixing part can be formed, for example, from a resin as a sealant, with the resin being one that is not cytotoxic, and is not degenerated or decomposed through contact with sterilizing, cleaning, or culture solutions. Examples of such a resin are curable resins such as polyurethane type resins, silicone type resins, epoxy resins, polysulfide type resins, and acrylic type resins.

The hollow fibers pass through the hollow fiber fixing part in a way such that the hollow fibers communicate with the inside of the flow tube and liquid will not leak.

For example, using a plurality of hollow fibers, the hollow fiber fixing part can be formed as follows. The open ends of the hollow fibers are lined up with one another and the hollow fibers are bundled together using a sealant. Next, the resulting bundle of hollow fibers is inserted into a resin tube having an outside diameter the same as the inside diameter of the flow tube, and in this state gaps between the bundle of hollow fibers and the resin tube are filled up with the sealant. Next, the hollow fibers are cut together with the resin tube, thus exposing an opening part of each hollow fiber. The resin tube having the hollow fibers fixed therein is then inserted into the flow tube with the open ends of the hollow fibers pointing into the flow tube, and the flow tube and the resin tube are sealed together using the sealant.

### Centrifuging vessel

The hollow fiber-possessing instrument of the present invention can be made to further have a centrifuging vessel having a size such that the hollow fibers can be disposed therein. 'A size such that the hollow fibers can be disposed therein' includes not only the case of a size such that the hollow fibers can be disposed in the centrifuging vessel without being bent, but also a size such that the hollow fibers can be disposed in the centrifuging vessel with the hollow fibers bent to an extent such as not to break. The centrifuging vessel generally comprises a main body and a lid, with the lid being supported by the flow tube. In the case that the flow tube comprises an inflow side flow tube and an outflow side flow tube, it is preferable for the lid of the centrifuging vessel to be supported by the inflow side flow tube. During centrifuging, by closing the lid onto the main body of the centrifuging vessel, the hollow fibers can be subjected to centrifuging using a centrifuge as is in a state disposed inside the main body.

Through the hollow fiber-possessing instrument of the present invention having a centrifuging vessel, after cells have been injected into the hollow fibers, centrifugal force can easily be applied to form cell aggregates as described later. Moreover, when injecting the cell suspension into the hollow fiber lumens as well, the injection operation can be carried out easily with the hollow fibers disposed inside the centrifuging vessel.

### (11) Methods of using the hollow fiber-possessing instrument of the present invention

A first method of using the hollow fiber-possessing instrument of the present invention is a method comprising injecting a cell suspension from the open end of the flow tube to accumulate cells in the hollow fiber lumens, and then culturing or storing the cells in a state in which the hollow fibers having the cells accumulated therein are immersed in a cell culture solution or a solution for cell storage.

A second method of using the hollow fiber-possessing instrument of the present invention is a method comprising injecting a cell suspension from the open end of the flow tube to accumulate cells in the hollow fiber lumens, applying centrifugal force to the cells in the hollow fiber lumens in a state in which the hollow fibers are disposed in a centrifuging vessel to form cell aggregates, and then culturing or storing the cells in a state in which the hollow fibers holding the cell aggregates are immersed in a cell culture solution or a solution for cell storage.

### Pretreatment

It is preferable to subject the hollow fibers to hydrophilic treatment before using the hollow fiber-possessing instrument of the present invention, so that the culture solution will be able to pass smoothly through the hollow fiber lumens. The hydrophilic treatment can be carried out, for example, by injecting in ethanol diluted with water to approximately 70%, and then water, from the opening part of the flow tube using an injection syringe or the like.

Next, the hollow fiber-possessing instrument of the present invention is generally sterilized. The sterilization can be carried out, for example, by subjecting the hollow fiber-possessing instrument to high-pressure steam sterilization. In the case that the flow tube comprises an inflow side flow tube and an outflow side flow tube, the two can be sterilized in a state detached from one another. As a result, it is possible, for example, to subject the inflow side flow tube to ordinary high-pressure steam sterilization, and subject the outflow side flow tube having the hollow fibers to high-pressure steam sterilization while immersed in distilled water.

### Injecting in of cell suspension

Before injecting in the cell suspension, it is preferable to fill the hollow fiber lumens with a liquid medium for cell culture, a solution for cell storage such as a solution for organ storage, a suitable buffer or the like. In this case, the liquid culture medium or the like can be filled into the hollow fiber lumens by injecting the liquid culture medium or the like in from the opening part of the flow tube using an injection syringe or the like, or alternatively the liquid culture medium or the like can be filled into the hollow fiber lumens by applying suction to the inside of the flow tube using an injection syringe or the like in a state in which the hollow fibers are immersed in the liquid culture medium or the like.

Next, a cell suspension that has been prepared by suspending cells in a liquid medium for cell culture, a solution for cell storage, a suitable buffer or the like is injected into the hollow fiber lumens from the opening part of the flow tube. The cell suspension is preferably prepared using the liquid culture medium, solution for cell storage or buffer that was filled into the hollow fiber lumens in advance. It is particularly preferable to use a liquid culture medium having the same composition.

As a liquid medium for cell culture, one suited to the cells should be selected as appropriate. For example, it is possible to use a basal medium such as Dulbecco's modified eagle medium, an RPMI-1640 medium, or Ham's F-12 medium to which have been added factors necessary for the growth of the cells in question such as growth factors and serum.

As a solution for cell storage, for example a solution for organ storage such as a Euro-Collins solution, a Ficoll-Collins solution, a UW solution (Japanese Patent Publication No. H7-68082), or a raffinose solution can be used.

The injecting in of the cell suspension can be carried out, for example, using an injection syringe, a micropipette, an installment injector or the like, although there is no limitation thereto. When the cell suspension is injected in from the opening part of the flow tube, because the tip of each hollow fiber is sealed, the liquid culture medium or the like flows out from the pores of each hollow fiber, and thus cells are accumulated in the lumen of each hollow fiber. The density of cells accumulated in the hollow fiber lumens varies according to the size of the cells, the inside diameter of the hollow fibers, and so on, but for example in the case of hepatocytes is generally approximately 1×10⁷ to 8×10⁷ cells/ml.

In the case of using a hollow fiber-possessing instrument provided with a centrifuging vessel, and injecting in the cell suspension with the hollow fibers disposed inside the centrifuging vessel, it is preferable to inject in the cell suspension with the lid of the centrifuging vessel loosened. As a result, the injection operation can be carried out easily and without soiling other vessels.

### Application of centrifugal force

After the cells have been injected into the hollow fiber lumens, a centrifuging vessel is attached to the hollow fiber-possessing instrument, and centrifuging is carried out using a centrifuge on the hollow fiber-possessing instrument complete with the centrifuging vessel, whereby centrifugal force can be applied to the cells in the hollow fiber lumens. In this case, it is preferable to apply the centrifugal force the cells in a state in which the hollow fibers have been immersed in a liquid culture medium or the like by filling the inside of the centrifuging vessel with the liquid culture medium or the like. As a result, the cells are not exposed to a gas phase during the centrifuging, and hence damage to the cells is reduced. By applying the centrifugal force, cell aggregates can easily be formed in the hollow fiber lumens.

In the present invention, 'cell aggregate' refers to a state in which the cells are adhered together to a high degree or with a high frequency to the extent that this would not be attainable when dispersed cells aggregate spontaneously.

There are no particular limitations on the centrifugal force so long as the cell aggregates can be formed; the centrifugal force should be determined within a range such that the cells are not damaged in accordance with the type of cells. The centrifugal force varies according to the type of cells, but is preferably made to be approximately 2 to 2,000×G, particularly preferably approximately 4 to 500×G. For example, in the case of primary rat hepatocytes, it is generally preferable to make the centrifugal force be 1,500×G or less, particularly preferably 400×G or less. In the case of primary rat hepatocytes, there are no particular limitations on the lower limit of the centrifugal force so long as the cell aggregates can be formed, but this lower limit is generally approximately 5×G. The centrifuging time should be set as appropriate within a range such that the cell aggregates can be formed. For example, with rat hepatocytes, by applying centrifugal force of approximately 50×G for approximately 90 seconds, cell aggregates having a density of approximately 5×10⁷ to 1×10⁸ cells/ml can be obtained.

### Culture of cells

After the cells have been injected into the hollow fiber lumens, or after centrifugal force has been further applied to form cell aggregates in the hollow fiber lumens, the cells can be subjected to culture. In this case, the cells can be cultured with the hollow fiber-possessing instrument holding the cells as is by immersing the hollow fiber part in a culture solution, or alternatively the cells can be cultured by cutting off the hollow fibers and then immersing the hollow fibers in a culture solution. In particular, to carry out the culture under conditions that are suitable for the cells, and so that the culturing operation is easy, it is preferable to cut the hollow fibers off, and then transfer the hollow fibers into a commonly used culture vessel and culture the cells. Unlike a conventional hollow fiber-possessing culture instrument in which the hollow fibers are fixed in a housing, the instrument of the present invention has a structure according to which the hollow fiber part can be easily exposed, i.e. the required hollow fiber part can be easily cut off, and hence culture of the cells in the hollow fibers can easily be carried out. The culture temperature varies according to the type of the cells, but is generally preferably made to be approximately 37°C.

For example, if primary rat hepatocytes are injected into the hollow fiber lumens, centrifugal force is applied to form cell aggregates in the hollow fiber lumens, and then the hollow fiber bundle is cut off to a length of approximately 30mm using a scalpel or the like, then it is possible to carry out culture by putting the hollow fibers holding the cells into a 35mm-diameter culture dish commonly used in cell culture. In the case of using a bundle of six hollow fibers having an inside diameter of 290µm, the total number of cells in the hollow fiber lumens is approximately 5×10⁵.

Moreover, by squashing the cut end part of the hollow fibers using tweezers or the like, the cells in the hollow fiber lumens can be prevented from leaking out.

### Storage of cells

After the cells have been injected into the hollow fiber lumens, or after cell aggregates have further been formed, the cells can be stored at normal temperature in a state in which the hollow fibers are immersed in a liquid medium for cell culture or a solution for cell storage such as a solution for organ storage. In this case, the storage can be carried out with the hollow fiber-possessing instrument holding the cells as is by immersing the hollow fiber part in a solution for storage, or alternatively the storage can be carried out by cutting off the hollow fibers and then immersing the hollow fibers in a solution for storage. In particular, to improve the storage efficiency, and so that handling is easy, it is preferable to cut the hollow fibers off and then carry out the storage. With the instrument of the present invention, the hollow fibers can easily be cut off.

The storage can be carried out in an open vessel, or can be carried out in a hermetically sealed vessel. In the case of primary rat hepatocytes, storage can be carried out in the state of cell aggregates for up to at least approximately 5 days with the various functions of the cells maintained as is, although this varies according to the type of the solution for storage.

Preferable embodiments of the present invention will now be further described with reference to the drawings.

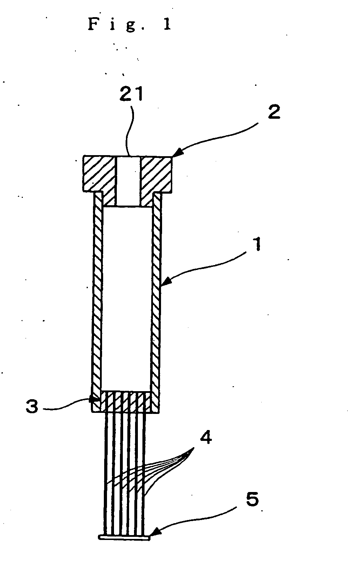

FIG. 1 is a sectional view of a hollow fiber-possessing instrument according to one embodiment of the present invention. The hollow fiber-possessing instrument has a cylindrical cell suspension flow tube 1, and into an open end of the flow tube 1 is fitted an adapter 2 for fitting to a discharge port of a cell injecting instrument (here, a disposable injection syringe, not shown in Fig.1). Moreover, a hollow fiber fixing part 3 formed from a curable resin is fitted into the other end of the flow tube 1. A bundle of hollow fibers 4 passes through the fixing part 3, and each of the hollow fibers 4 communicates with the inside of the flow tube 1. Moreover, the other end of each of the hollow fibers 4 is sealed with a curable resin 5.

When using this hollow fiber-possessing instrument, the hollow fibers 4 of the hollow fiber-possessing instrument, which have been subjected in advance to hydrophilic treatment and then to autoclave sterilization while immersed in distilled water, are immersed in a liquid culture medium or the like, and in this state a discharge port of an injection syringe, not shown, is fitted to an injection port 21 of the adapter 2 and suction is carried out. As a result, the liquid culture medium or the like is filled into the hollow fibers 4 and the inside of the flow tube 1. Next, a cell suspension is injected in from the injection port 21 using an injection syringe. The cell suspension passes through the lumen of the flow tube 1, and enters the lumen of each hollow fiber 4 from an opening part of the hollow fiber 4; the liquid culture medium flows out from the pores of each hollow fiber 4, whereby cells are accumulated in the lumen of each hollow fiber 4.

After filling with the cells, the hollow fibers 4 are cut to a suitable length, and are immersed in a separately prepared liquid culture medium, whereby culture can be carried out.

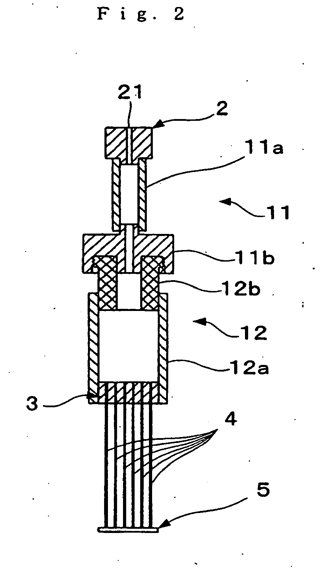

FIG. 2 is a sectional view of a hollow fiber-possessing instrument according to another embodiment of the present invention. This hollow fiber-possessing instrument is like the hollow fiber-possessing instrument of FIG. 1, but the flow tube 1 comprises an inflow side flow tube 11 and an outflow side flow tube 12 that are detachably coupled together. The inflow side flow tube 11 comprises a tube 11a made of a flexible material (e.g. a silicone) and a male-shaped connector 11b that is coupled to the tube 11a Moreover, the outflow side flow tube 12 comprises a tube 12a made of a flexible material (e.g. a silicone) and a female-shaped connector 12b that is coupled to the tube 12a. A hollow fiber fixing part 3 is formed at the opposite end of the tube 12a to the end to which the connector 12b is connected. Here, the male-shaped connector 11b and the female-shaped connector 12b are able to be screwed together. The rest of the constitution is the same as for the hollow fiber-possessing instrument of FIG. 1, with corresponding components being given the same reference numeral.

When using this hollow fiber-possessing instrument, the flow tube 1 is separated into the outflow side flow tube 12 to which the hollow fibers 4 are connected and the inflow side flow tube 11, whereby the outflow side flow tube 12 and the inflow side flow tube 11 can be sterilized separately. The connector 11b and the connector 12b are then coupled together, and then the hollow fiber-possessing instrument is used as with the hollow fiber-possessing instrument of FIG. 1.

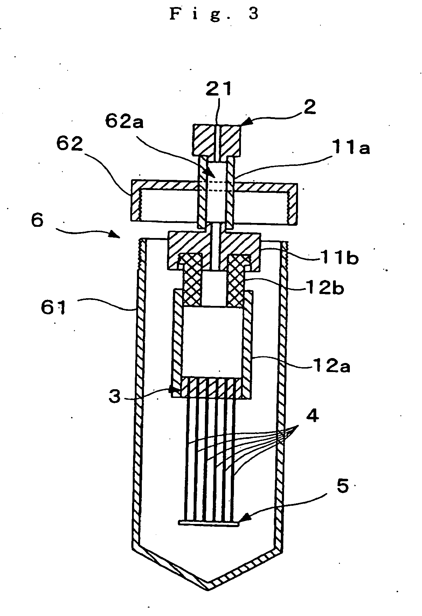

FIG. 3 is a sectional view of a hollow fiber-possessing instrument according to yet another embodiment of the present invention. This hollow fiber-possessing instrument is like the hollow fiber-possessing instrument of FIG. 2, but further has a centrifuging vessel 6. The centrifuging vessel 6 comprises a main body 61 and a lid 62; the tube 11a of the inflow side flow tube 11 of the flow tube 1 passes through a hole 62a provided in the lid 62, and in this state the lid 62 is fixed to the tube 11a Moreover, the main body 61 of the centrifuging vessel 6 has a volume such that the hollow fibers 4 can be disposed therein.

When using this hollow fiber-possessing instrument, cells are filled into the hollow fiber lumens as in the case of the hollow fiber-possessing instrument of FIG. 1 or 2, the hollow fibers 4 are put into the main body 61 of the centrifuging vessel 6 and the lid 62 is closed, and centrifuging is carried out using a centrifuge. Moreover, after filling in the cells but before applying the centrifugal force, a cap, not shown, may be put over the injection port 21, whereby microbial contamination of the cells can be prevented.

### Best Mode For Carrying Out The Invention

Following is a detailed description of the present invention through examples; however, the present invention is not limited to these examples.

### Manufacturing Example 1A

### Manufacture of hollow fiber-possessing instrument

Hollow fibers for blood plasma separation made of cellulose triacetate (AP250N15 type Toyobo Co., Ltd.; inside diameter 285µm, outside diameter 387µm, membrane thickness 51µm, pore size 0.2µm) were used as the hollow fibers. One end of each of six such hollow fibers of length approximately 7cm was sealed by silicone bonding to form a sealed part 5. The other end was bonded by silicone bonding to bundle the hollow fibers together, and then the bundle was inserted into a sealing part silicone tube of outside diameter 4mm. Next, cutting was carried out with a scalpel, thus forming an opening part in each hollow fiber 4. The sealing part silicone tube in which the hollow fibers had been fixed was fitted into a silicone tube of inside diameter 4mm and length 15mm used as an outflow side flow tube 12a, and gaps between the two tubes were filled up with a silicone resin. Next, the silicone tube 12a was connected to a female-shaped connector 12b ( Isis). Coupling was then carried out to a separately prepared part comprising a silicone tube for an inflow side flow tube 11a of inside diameter 2mm and length 20mm and a male-shaped connector 11b ( Isis). The inflow side flow tube 11a silicone tube was passed through a hole provided in a lid of a centrifuging tube of inside diameter 14mm and length 120mm ( Falcon) and fixed to the lid.

Using an injection syringe, 3ml of 70% ethanol and then 10ml of distilled water were sucked in from the opening part side of the hollow fiber 4, thus making the lumen of each hollow fiber 4 hydrophilic. Next, uncoupling was carried out at the connector part, and the outflow side part and the inflow side part were separately sterilized in an autoclave. The outflow side part was sterilized with the hollow fibers immersed in distilled water. The separately sterilized parts were then connected together at the connector part.

### Example 1A: Culture of cell aggregates

10ml of a serum-free medium (HSFM) comprising 13.5g/l of Dulbecco's modified eagle medium (DMEM; Gibco), 60mg/1 of proline, 50ng/ml of EGF (Toyobo Co., Ltd.), 10mg/1 of insulin (Sigma), 7.5mg/l of hydrocortisone (Sigma), 50µg/1 of linoleic acid (Sigma), 100,000 U/l of penicillin G potassium (Nacalai Tesque, Inc.), 100mg/1 of streptomycin sulfate (Nacalai Tesque, Inc.), 1.05g/l of sodium hydrogencarbonate (Nacalai Tesque, Inc.) and 1.19g/l of HEPES (Nacalai Tesque, Inc.) was put into a centrifuging tube. The hollow fiber bundle of a hollow fiber-possessing instrument manufactured as described above was immersed in the HSFM, and the HSFM was sucked in using an injection syringe, thus eliminating air bubbles from the hollow fiber lumens and also replacing the water in the hollow fiber lumens with HSFM.

Primary rat hepatocytes that had been prepared using a collagenase digestion method were suspended in the above-mentioned HSFM to form a suspension, and a total of 1.0x10⁷ cells were injected in from the injection port using an injection syringe. After injecting in the cells, the injection port was capped, and centrifuging was carried out on the hollow fiber-possessing instrument complete with the centrifuging tube using a centrifuge, with a centrifugal force of 60xG being applied to the cells for 90 seconds, whereby cell aggregates were formed in the hollow fiber lumens.

Next, the part on the hollow fiber bundle side was taken off at the connector part, the hollow fibers were cut off at a place 3cm from the tip part where the hollow fibers were sealed, and then the hollow fibers were immediately put into a 35mm-diameter dish ( Iwaki Glass) into which had been put 2ml of HSFM, and culture was carried out while shaking under an atmosphere of 5% carbon dioxide and 95% air.

### Comparative Example 1A: Monolayer culture

For comparison, 5.0×10⁵ of the same primary rat hepatocytes as in Example 1A were seeded into a collagen-coated 35mm-diameter dish ( Iwaki Glass), and monolayer culture was carried out in 2ml of the same HSFM as in Example 1A under the same conditions as in Example 1A.

### Test Example 1A: Evaluation of ammonia metabolizing capability and albumin producing ability

Ammonia was added to the HSFM of each of Example 1A and Comparative Example 1A such that the ammonia concentration was 1 mM, and the ammonia metabolizing capability was measured by measuring the ammonia concentration over time using an automatic analyzer. Moreover, the albumin producing ability was measured by measuring the concentration of albumin secreted into the HSFM over time by an EIA method using an anti-rat albumin antibody.

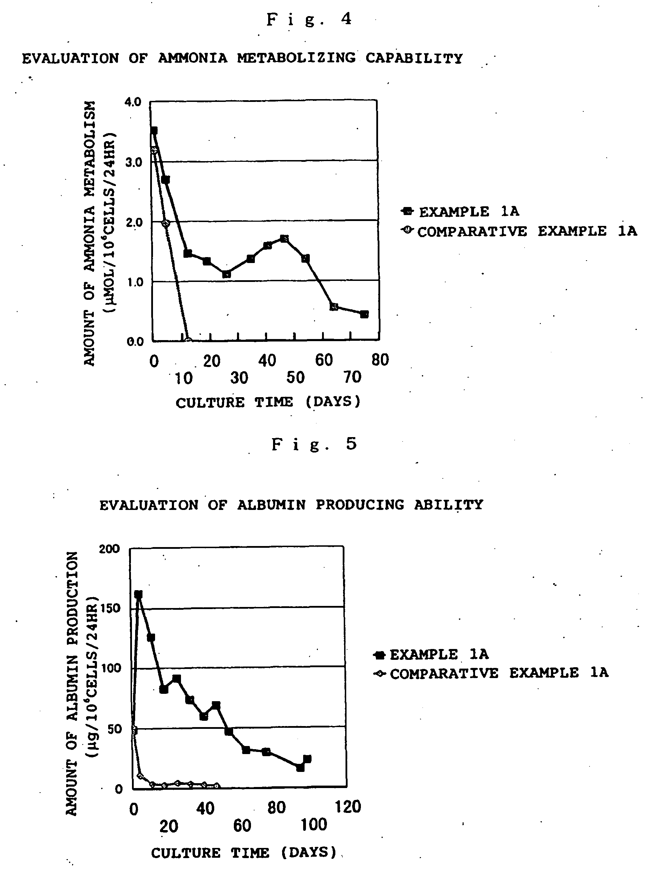

The ammonia metabolizing capability measurement results are shown in FIG. 4, and the albumin producing ability measurement results are shown in FIG.5. As is clear from FIGS. 4 and 5, for the cell aggregates of Example 1A, the ammonia metabolizing capability and the albumin producing ability were both maintained for a prolonged period of over 2 months, although these abilities did drop over time compared with initially. On the other hand, for the monolayer culture of Comparative Example 1A, although the initial activity was similar to that of the cell aggregates of Example 1A, the activity was quickly lost, i.e. within 10 days of commencing the culture.

It can be seen that by culturing cells using the hollow fiber-possessing instrument of the present invention, the cells can be cultured with the functions of the cells maintained over a prolonged period. That is, it can be seen that the hollow fiber-possessing instrument of the present invention is suitable for use in cell storage or cell culture for carrying out cell function tests, metabolizing capability tests, tests on the production of substances by cells, or the like.

### Example 1B

5×10⁵ primary rat hepatocytes that had been prepared using a collagenase digestion method were injected into the lumens of hollow fibers for blood plasma separation made of cellulose triacetate (AP250N15 type made by Toyobo Co., Ltd.; inside diameter 285 µm, outside diameter 387µm, pore size 0.4µm), and a centrifugal force of 60×G was applied for 90 seconds, thus forming cell aggregates in the hollow fiber lumens.

Six such hollow fibers of length 3 cm having hepatocyte aggregates filled therein were put, in a state with both ends sealed, into a 35mm-diameter culture dish ( Falcon), and tissue body formation was induced by carrying out culture with shaking for 2 days on a shaker under an atmosphere of 5% carbon dioxide and 95% air in a serum-free medium (HSFM) obtained by adding 60 mg/l of proline, 50 ng/ml of EGF (Toyobo Co., Ltd.), 10 mg/l of insulin (Sigma), 7.5 mg/l of hydrocortisone (Sigma), 50 µg/1 of linoleic acid (Sigma), 100,000 U/l of penicillin G potassium (Nacalai Tesque, Inc.), 100 mg/l of streptomycin sulfate (Nacalai Tesque, Inc.), 1.05 g/l of sodium hydrogencarbonate (Nacalai Tesque, Inc.) and 1.19 g/l of HEPES (Nacalai Tesque, Inc.) to 13.5 g/l of Dulbecco's modified eagle medium (DMEM; Gibco).

Next, the tissue bodies were subjected to a 72-hour transportation test. Specifically, the hollow fibers having the hepatocyte tissue bodies formed in the lumens thereof were transferred from the culture dish into a 15 ml centrifuging tube filled with HSFM, the centrifuging tube was sealed and packaged, and then the centrifuging tube was transported by truck for 72 hours (approximately 1,200 km) at room temperature. After the transportation, the hollow fibers were put back into a culture dish, and culture was restarted under the same conditions as before the transportation.

### Example 2B

Culture was carried out as in Example 1B, and without carrying out a transportation test, the culture was continued as is in the culture dish, with the HSFM medium being replaced at 1-day intervals.

### Comparative Example 1B

2.0×10⁶ primary rat hepatocytes that had been prepared as in Example 1B were seeded into a flask (Falcon) having a culture area of 25 cm², and monolayer culture was carried out for 2 days under an atmosphere of 5% carbon dioxide and 95% air using HSFM as in Example 1B. After that, the flask was filled with HSFM and was sealed up and packaged, and was then subjected to a 72-hour truck transportation test as in Example 1B.

### Comparative Example 2B

Monolayer culture was carried out as in Comparative Example 1B, and without carrying out a transportation test, the culture was continued as is in the flask, with the HSFM medium being replaced at 1-day intervals. :

### Test Example 1B: Evaluation of cell functions

Hepatocytes have an ability to synthesize proteins such as albumin, and also a function of metabolizing ammonia, drugs and so on, but maintaining these functions during storage is difficult. Here, the amount of albumin production and the amount of ammonia metabolism were measured over time.

For the samples of Example 1B and Comparative Example 1B, measurements were carried out 1 day after commencing the culture, 1 day after recommencing the culture (i.e. 6 days after first commencing the culture), 8 days after recommencing the culture (i.e. 13 days after first commencing the culture), and 15 days after recommencing the culture (i.e. 20 days after first commencing the culture). For the samples of Example 2B and Comparative Example 2B, measurements were carried out 1 day after commencing the culture, 6 days after commencing the culture, 13 days after commencing the culture, and 20 days after commencing the culture.

The amount of albumin in the culture medium was measured by an EIA method using an anti-rat albumin, and the amount of ammonia metabolism was determined by using an automatic analyzer to measure the amount of consumption of ammonia that had been loaded into the culture medium.

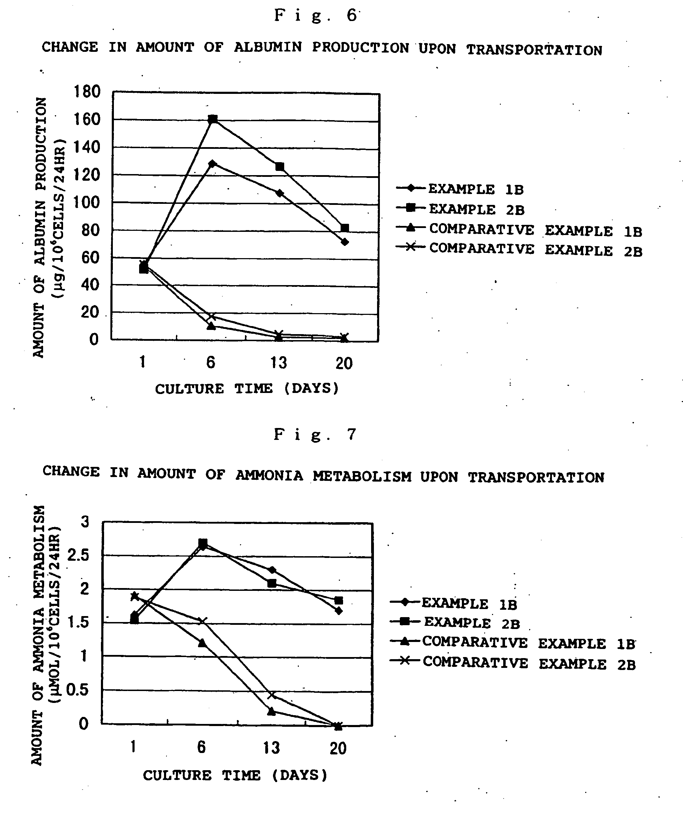

The albumin production amount measurement results are shown in FIG. 6, and the ammonia metabolism amount measurement results are shown in FIG. 7. As is clear from FIGS. 6 and 7, for the cells of Example 2B in which culture was carried out after forming aggregates of the hepatocytes in hollow fibers, a high albumin producing ability and a high ammonia metabolizing capability were exhibited and maintained. It is thought that the reason that the albumin producing ability and the ammonia metabolizing capability both exhibited higher values after 6 days than initially is that tissue body formation was induced by the culturing, and hence the manifestation of the functions was enhanced.

Moreover, for Example 1B in which the cells were transported for 3 days, even though transportation was carried out, the albumin producing ability and the ammonia metabolizing capability were maintained at approximately the same level as in Example 2B. It is conjectured that this is because in Example 1B tissue body formation proceeded to some extent during the transportation.

In contrast, for the cells of Comparative Examples 1B and 2B in which monolayer culture was carried out, no difference to the cells of Examples 1B and 2B was seen initially, but as the number of days of culture passed the functions of the cells were lost rapidly. For Comparative Example 1B in which transportation was carried out, the loss of functions was even more rapid.

### Example 1C

5×10⁵ primary rat hepatocytes that had been prepared using a collagenase digestion method were filled by centrifuging at 60×G for 90 seconds into the lumens of six hollow fibers for blood plasma separation made of cellulose triacetate (AP250N15 type made by Toyobo Co., Ltd.; inside diameter 285 µm, outside diameter 387 µm) that had had tip parts thereof sealed by silicone bonding and had been bundled together. After the hepatocytes had been filled in, a part 3cm from the tips of the hollow fibers was compression bonded, and this part was cut, whereby a bundle of 3cm-long hollow fibers having hepatocytes sealed therein was obtained.

The hollow fiber bundle was put into a 35mm-diameter cell culture petri dish (Falcon), and 2 ml of a serum-free medium (HSFM) that had been obtained by adding 60 mg/l of proline, 50 ng/ml of EGF (Toyobo Co., Ltd.), 10 mg/l of insulin (Sigma), 7.5 mg/l of hydrocortisone (Sigma), 50 µg/L of linoleic acid (Sigma), 100,000 U/l of penicillin G potassium (Nacalai Tesque, Inc.), 100 mg/l of streptomycin sulfate (Nacalai Tesque, Inc.), 1.05 g/l of sodium hydrogencarbonate (Nacalai Tesque, Inc.) and 1.19 g/l of HEPES (Nacalai Tesque, Inc.) to 13.5 g/l of Dulbecco's modified eagle medium (DMEM; Gibco) was added.

The petri dish was put onto a shaker (Iwaki Glass), and culture with rotation and shaking was carried out under conditions of a shaking amplitude of 25mm and a rotational speed of 45 rpm at 37°C under an atmosphere of 5% carbon dioxide and 95% air.

### Comparative Example 1C

A bundle of hollow fibers having hepatocytes sealed therein was obtained as in Example 1C. The hollow fiber bundle was put into a 35mm-diameter cell culture petri dish, 2ml of HSFM was added, and stationary culture was carried out at 37°C under an atmosphere of 5% carbon dioxide and 95% air.

### Comparative Example 2C

1.0×10⁶ primary rat hepatocytes that had been prepared as in Example 1C were suspended in HSFM to which had been added 5% fetal bovine serum, and seeding was carried out into a collagen-coated 35mm-diameter petri dish. After the cells had adhered, the culture medium was replaced with 2ml of HSFM, and then stationary culture was carried out at 37°C under an atmosphere of 5% carbon dioxide and 95% air.

### Test Example 1C: Evaluation of cell functions

Hepatocytes have an ability to synthesize proteins such as albumin, and also a function of metabolizing ammonia, drugs and so on. It is known that the ammonia metabolizing capability in particular is generally lost rapidly as the number of days of culture passes. Here, the amount of albumin production and the amount of ammonia metabolism were measured over time.

For the cell cultures of each of Example 1C, Comparative Example 1C and Comparative Example 2C, the amount of albumin production and the amount of ammonia metabolism were measured 1 day, 3 days, 7 days, 14 days, 21 days and 28 days after commencing the culture.

The amount of albumin production was determined by measuring the amount of albumin in the culture medium by an EIA method using an anti-rat albumin, and the amount of ammonia metabolism was determined by using an automatic analyzer to measure the amount of consumption of ammonia that had been loaded into the culture medium.

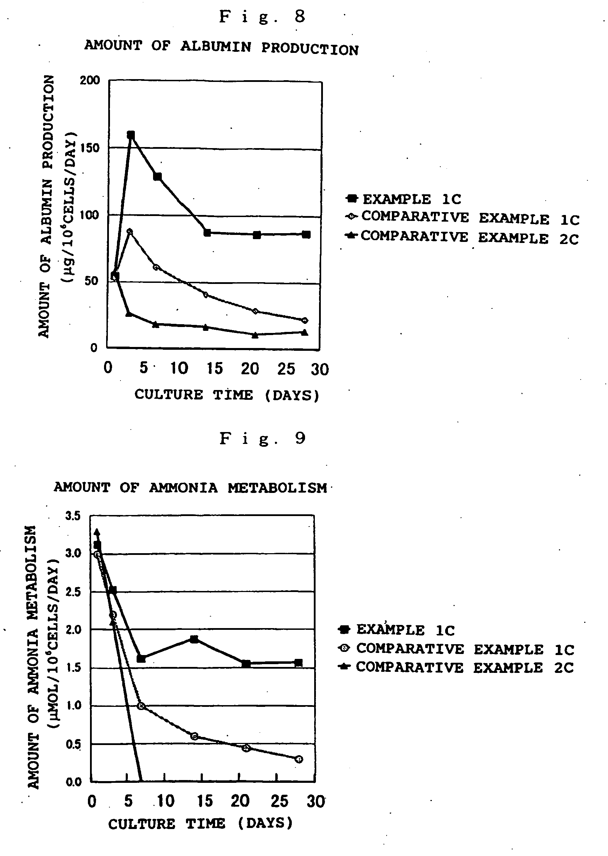

The albumin production amount measurement results are shown in FIG. 8, and the ammonia metabolism amount measurement results are shown in FIG. 9. As is clear from FIGS. 8 and 9, for the cells of Example 1C in which aggregates of the hepatocytes were formed in the hollow fiber lumens using centrifugal force and then culture with shaking was carried out, a high albumin producing ability and a high ammonia metabolizing capability were exhibited and maintained.

In contrast, for the cells of Comparative Example 1C in which stationary culture was carried out and Comparative Example 2C in which stationary monolayer culture was carried out, no difference to the cells of Examples 1C and 2C was seen initially, but the functions of the cells were lost rapidly as the number of days of culture passed.

### Example 1D

Primary human cartilage cells that had been cultured in a flask were subjected to trypsin digestion, thus preparing a cell dispersion containing 7.5×10⁶ cells/ml. The cell dispersion was injected into the lumens of hollow fibers for blood plasma separation made of cellulose triacetate (AP250N15 type made by Toyobo Co., Ltd.; inside diameter 285 µm, outside diameter 387 µm), and then a centrifugal force of 60×G was applied for 90 seconds, thus forming cell aggregates in the hollow fiber lumens.

Six such hollow fibers of length 3cm having cartilage cell aggregates sealed therein were prepared, and were put into a 35mm-diameter culture dish (Falcon), and differentiation was induced by carrying out culture with shaking for 1 month at 37°C on a shaker under an atmosphere of 5% carbon dioxide and 95% air in a differentiation-inducing culture medium obtained by adding 5% fetal bovine serum, insulin (Sigma), TGF-β, transferrin, penicillin G potassium (Nacalai Tesque, Inc.), streptomycin sulfate (Nacalai Tesque, Inc.) and sodium hydrogencarbonate (Nacalai Tesque, Inc.) to Dulbecco's modified eagle medium (DMEM; Gibco).

The cells in the hollow fibers were subjected to formalin fixation and paraffin embedding after varying numbers of days, thin sections were prepared, and toluidine blue staining was carried out, whereby the state of cell differentiation could be investigated.

### Comparative Example 1D

The human cartilage cell dispersion prepared in Example 1D was diluted with a differentiation-inducing culture medium such that the concentration of cells became 0.2×10⁶ cells/ml, seeding into a flask (Falcon) having a culture area of 25 cm² was carried out, 5 ml of a differentiation-inducing culture medium like that used in Example 1D was put in, and monolayer culture was carried out under the same environment as in Example 1D. The cells were subjected to methanol fixation after varying numbers of days, toluidine blue staining was carried out, and observation was carried out under a microscope.

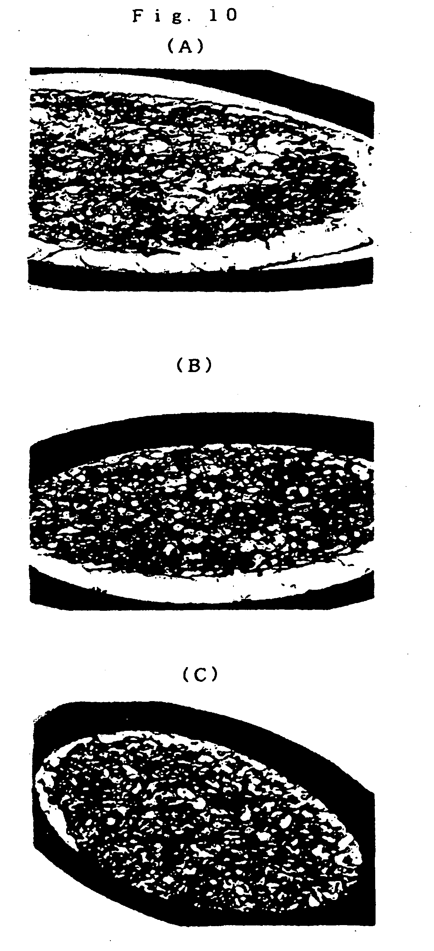

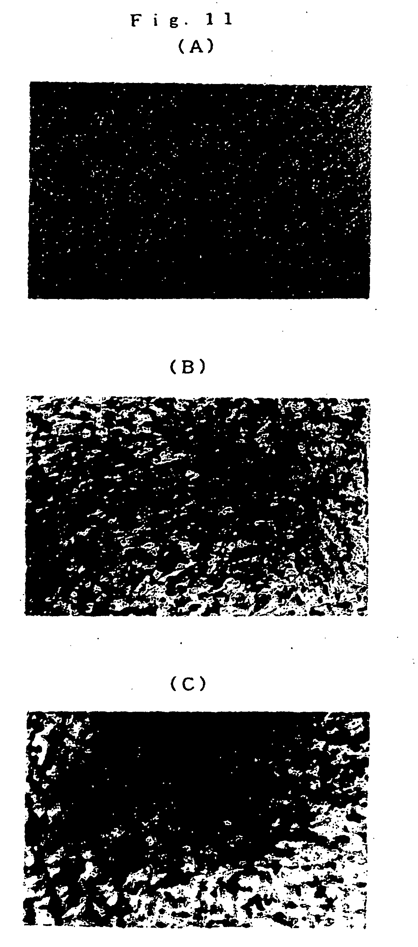

The toluidine blue stained images of the cells observed for Example 1D and Comparative Example 1D are shown in FIGS. 10 and 11 respectively. In FIGS. 10 and 11, (A) shows the state 1 week after commencing the culture, (B) shows the state 2 weeks after commencing the culture, and (C) shows the state 4 weeks after commencing the culture.

As is clear from FIG. 10, for the cartilage cell culture in the hollow fibers obtained in Example 1D, 1 week after commencing the differentiation-inducing culture (A), toluidine blue metachromasia indicating formation of a cartilage matrix was not seen, but from 2 weeks after commencing the culture (B), metachromasia started to be seen in the cells, and 4 weeks after commencing the culture (C), metachromasia was clearly seen throughout the whole of the cellular tissue body. It is thought that 4 weeks after commencing the culture, the tissue body was formed from a group of cells having homogeneous differentiated functions.

In Example 1D, cartilage cells that had not undergone final differentiation, i.e. cartilage cells having differentiation potential, approached a cartilage tissue form, which is a finally differentiated form, through the differentiation inducing method of the present invention.

In contrast, for the cartilage cell culture obtained through monolayer culture in Comparative Example 1D, as in Example 1D, 1 week after commencing the culture (A), toluidine blue metachromasia was not exhibited, but from 2 weeks after commencing the culture (B), metachromasia started to be seen in the cells, and 4 weeks after commencing the culture (C), cells exhibiting metachromasia were seen. However, even 4 weeks after commencing the culture, the cells exhibiting metachromasia were limited to parts where the cell density was high, and hence differentiation was not induced throughout all of the cultured cells.

### Example 2D

Primary human fat precursor cells that had been cultured in a flask were subjected to trypsin digestion, thus preparing a cell dispersion containing 7.5×10⁶ cells/ml. The cell dispersion was injected into the lumens of hollow fibers for blood plasma separation made of cellulose triacetate (AP250N15 type made by Toyobo Co., Ltd.; inside diameter 285 µm, outside diameter 387 µm), and then a centrifugal force of 60xG was applied for 90 seconds, thus forming cell aggregates in the hollow fiber lumens.

Six such hollow fibers of length 3 cm having human fat precursor cell aggregates sealed therein were prepared, and were put into a 35mm-diameter culture dish (Falcon), and differentiation was induced by carrying out culture with shaking at 37°C on a shaker under an atmosphere of 5% carbon dioxide and 95% air in a differentiation-inducing culture medium obtained by adding 2% fetal bovine serum, insulin, transferrin, triiodthyronine, epidermal growth factor, dexamethasone, indomethacin, penicillin G potassium, streptomycin sulfate and sodium hydrogencarbonate to Dulbecco's modified eagle medium (DMEM). After culturing for 2 weeks, the hollow fibers were washed with PBS (phosphate buffered saline), and then the amount of neutral fat accumulated in the cells was measured using a diagnostic reagent for neutral fat measurement.

### Comparative Example 2D

The human fat precursor cell dispersion prepared in Example 2D was diluted with a differentiation-inducing culture medium such that the concentration of cells became 0.2×10⁶ cells/ml, seeding into a 35mm-diameter culture dish was carried out, 5 ml of a differentiation-inducing culture medium like that used in Example 2D was put in, and monolayer culture was carried out under the same environment as in Example 1D. After culturing for 2 weeks, the cells were washed with PBS, and then the amount of neutral fat accumulated in the cells was measured using a diagnostic reagent for neutral fat measurement.

The amount of neutral fat accumulated in the cells is shown in FIG. 12 for Example 2D and Comparative Example 2D. As is clear from FIG. 12, the amount of neutral fat accumulated in the cells was markedly greater in Example 2D than in Comparative Example 2D. This suggests that differentiation from fat precursor cells into fat cells was carried out efficiently by the differentiation inducing method of the present invention.

### Example 1E

100 µl of a 1.8×10⁵ cells/100 µl rat hepatocyte suspension that had been prepared using a collagenase digestion method was put into each well of a permeable vessel having 24 wells and having a polycarbonate permeable membrane on a bottom surface of each well (trade name: Transwell, for 24-well plate, membrane pore size: 3.0 µm, culture area: 0.33 cm², Corning Costar). Next, after allowing the cells to settle naturally for 5 minutes, a centrifugal force of 60xG was applied for 90 seconds, whereby a cell aggregate was formed on each permeable membrane. The number of layers of cells in each aggregate was found to be approximately 5 to 20 upon cutting out the permeable membrane on which the layered aggregate lay, carrying out formalin fixation, and then preparing a paraffin embedded section and observing with a microscope.

The Transwell in which a hepatocyte aggregate had been formed on each permeable membrane was placed as is on a 24-well cell culture plate (Falcon) such that the wells of the Transwell fitted into the wells of the cell culture plate.

Next, 650 µl of a serum free medium (HSFM) that had been obtained by adding 60 mg/l of proline, 50 ng/ml of EGF (Toyobo Co., Ltd.), 10 mg/l of insulin (Sigma), 7.5 mg/l of hydrocortisone (Sigma), 50 mg/L of linoleic acid (Sigma), 100,000 U/l of penicillin G potassium (Nacalai Tesque, Inc.), 100 mg/l of streptomycin sulfate (Nacalai Tesque, Inc.), 1.05 g/l of sodium hydrogencarbonate (Nacalai Tesque, Inc.) and 1.19 g/l of HEPES (Nacalai Tesque, Inc.) to 13.5 g/l of Dulbecco's modified eagle medium (DMEM; Gibco) was put into the wells of the cell culture plate, and stationaryculture was carried out for 1 month at 37°C under an atmosphere of 5% carbon dioxide and 95% air..

### Comparative Example 1E

Cells were cultured under the same conditions as in Example 1E, except that the centrifugal force was not applied.

### Comparative Example 2E

2.0x10⁶ primary rat hepatocytes prepared as in Example 1E were seeded into a flask (Falcon) having a culture area of 25cm², and monolayer culture was carried out for 1 month at 37°C under the same culture conditions as in Example 1E (an atmosphere of 5% carbon dioxide and 95% air) using the same medium (HSFM) as in Example 1E.

### Test Example 1E: Evaluation of cell functions

Hepatocytes have an ability to synthesize proteins such as albumin, and also a function of metabolizing ammonia, drugs and so on. It is known that the ammonia metabolizing capability in particular is generally lost rapidly as the number of days of culture passes. Here, the amount of albumin production and the amount of ammonia metabolism were measured over time.

For the samples of Example 1E, Comparative Example 1E and Comparative Example 2E, the amount of albumin production and the amount of ammonia metabolism were measured 1 day, 3 days, 7 days, 14 days, 21 days and 28 days after commencing the culture.

The amount of albumin production was determined by measuring the amount of albumin in the culture medium by an EIA method using an anti-rat albumin, and the amount of ammonia metabolism was determined by using an automatic analyzer to measure the amount of consumption of ammonia that had been loaded into the culture medium.

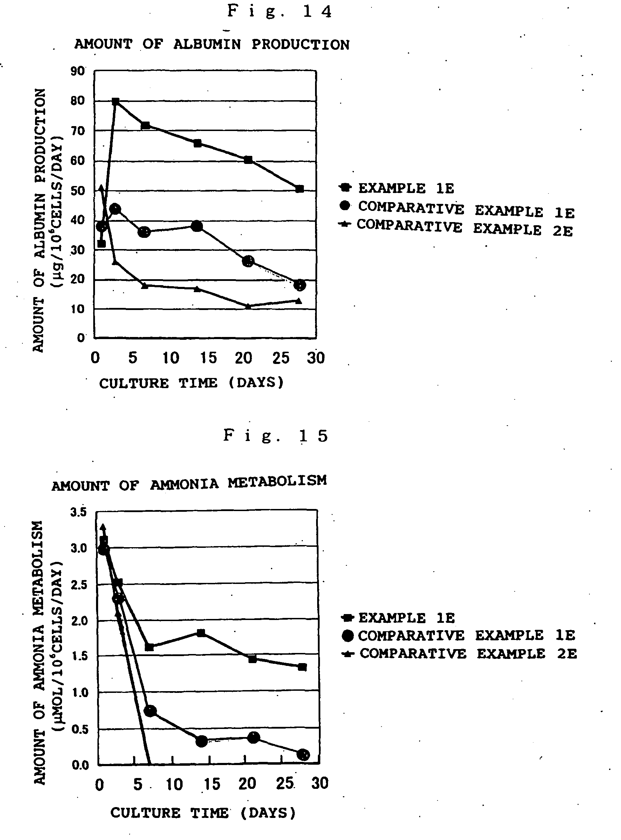

The albumin production amount measurement results are shown in FIG. 14, and the ammonia metabolism amount measurement results are shown in FIG. 15. As is clear from FIGS. 14 and 15, for the cells of Example 1E in which aggregates of the hepatocytes were formed on permeable membranes using centrifugal force and then culture was carried out, a high albumin producing ability and a high ammonia metabolizing capability were exhibited and maintained.

In contrast, for the cells of Comparative Example 1E in which the culture was carried out without carrying out centrifuging and Comparative Example 2E in which monolayer culture was carried out, no difference to the cells of Example 1E was seen initially, but the functions of the cells were lost rapidly as the number of days of culture passed.

### Example 1F

Primary human cartilage cells that had been cultured in a flask were subjected to trypsin digestion, thus preparing a cell dispersion containing 7.5×10⁶ cells/ml. The cell dispersion was injected into the lumens of hollow fibers for blood plasma separation made of cellulose triacetate (AP250N15 type made by Toyobo Co., Ltd.; inside diameter 285 µm, outside diameter 387 µm), and then a pressure (hydraulic pressure) of approximately 8 kg/cm² was applied using an injection syringe, thus forming cell aggregates in the hollow fiber lumens.

Six such hollow fibers of length 3cm having cartilage cell aggregates sealed therein were prepared, and were put into a 35mm-diameter culture dish (Falcon), and then a culture medium obtained by adding 5% fetal bovine serum, insulin (Sigma), TGF-β, transferrin, penicillin G potassium (Nacalai Tesque, Inc.), streptomycin sulfate (Nacalai Tesque, Inc.) and sodium hydrogencarbonate (Nacalai Tesque, Inc.) to Dulbecco's modified eagle medium (DMEM; Gibco) was further put into the culture dish, and culture with shaking was carried out for 1 month at 37°C on a shaker under an atmosphere of 5% carbon dioxide and 95% air.

RNA was extracted from the cells in the hollow fibers over time, and the type II collagen mRNA expression was investigated. Moreover, thin sections were prepared, and toluidine blue staining was carried out, whereby the state of cartilage cell matrix production could be investigated.

### Comparative Example 1F

The human cartilage cell dispersion prepared in Example 1F was diluted with a differentiation-inducing culture medium such that the concentration of cells became 0.2×10⁶ cells/ml, seeding into a flask (Falcon) having a culture area of 25cm² was carried out, 5ml of the same culture medium as that used in Example 1F was put in, and monolayer culture was carried out under the same environment as in Example 1F. RNA was extracted from the cells over time, and the type II collagen mRNA expression was investigated.

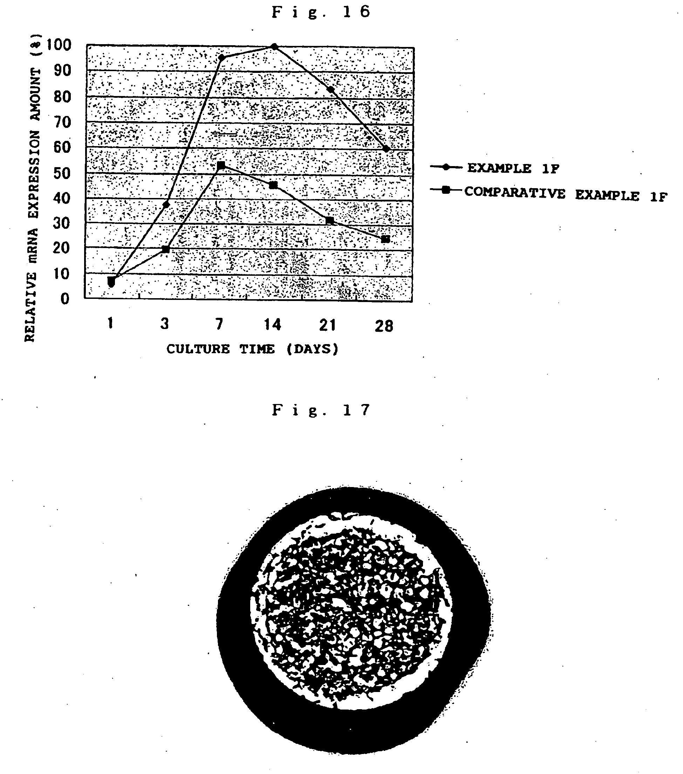

A graph showing a comparison of the relative amounts of type II collagen mRNA expression per unit number of cells for Example 1F and Comparative Example 1F is shown in FIG. 16.

As is clear from FIG. 16, the amount of mRNA expression for type II collagen, which is a cartilage differentiation marker, exhibited a higher value for the cartilage cells in the hollow fibers obtained in Example 1F than with the monolayer culture method of Comparative Example 1F over a prolonged period of time.

Moreover, in Example 1F, tissue-like bodies were formed in the hollow fibers. A tissue section was prepared, and toluidine blue staining was carried out; the results are shown in FIG. 17. As shown in FIG. 17, with the tissue section of Example 1F, metachromasia indicating cartilage matrix formation was seen strongly.

According to the present invention, there are provided a convenient hollow fiber-possessing instrument for culturing and storing cells inside hollow fibers, and a method of using the instrument.

The instrument of the present invention differs from conventional hollow fiber-possessing cell culture instruments, being small, and furthermore having an injection port to which can be fitted a discharge port of an injection syringe, a micropipette chip or the like that is widely used as an experimental instrument, so that cells can easily be injected into the hollow fiber lumens using an injection syringe or the like.

Moreover, unlike with conventional hollow fiber-possessing cell culture instruments, with the instrument of the present invention, the hollow fibers are not fixed inside a housing, and hence the hollow fibers can easily be exposed after injecting in cells; culture or storage of the cells in the hollow fiber lumens can thus be carried out easily merely by immersing the hollow fibers, either as or after being cut off, in a culture solution or a solution for cell storage after the cells have been injected in. In particular, one of the characteristic features of the instrument of the present invention is that the hollow fibers can easily be cut off, and hence can be moved into a vessel in which culture or storage can be carried out easily before being subjected to this culture or storage. Specifically, stable cell culture can be carried out easily in the hollow fiber lumens without a housing enveloping the hollow fibers, a refluxing apparatus or the like being required; as a result, it becomes possible to carry out experimental studies that make use of the high level of expression of cell functions over a prolonged period, which could not be obtained with a conventional culture system such as a monolayer culture system or a spheroid culture system.

Moreover, in the case that the hollow fiber-possessing instrument of the present invention is provided with a centrifuging vessel, centrifugal force can easily be applied after the cells have been injected into the hollow fiber lumens, and hence cell aggregates can easily be formed in the hollow fiber lumens.

In this way, the instrument of the present invention can be used easily, and hence is suitable for use in tests (experiments) for investigating the functions or metabolizing abilities of cells. Moreover, cells can also be stored easily in the hollow fiber lumens, and hence the instrument of the present invention is also suitable for use at a laboratory level.

According to the present invention, there are provided methods for seeding, culturing, storing, or inducing differentiation in cells with the functions of the cells maintained over a prolonged period.

By forming cell aggregates by applying centrifugal force, applying pressure or the like, and thus creating a state in which the frequency of contact between cells is greatly increased, cell aggregates in which the functions originally possessed by the cells are exhibited well can be obtained. Moreover, such aggregates can easily be induced into tissue bodies, with the tissue bodies formed from the cell aggregates exhibiting the functions originally possessed by the cells well.

With such cell aggregates or tissue bodies, the functions of the cells are not prone to being lost upon storing in a liquid medium for cell culture or a solution for organ storage, with the functions of the cells being exhibited well even after such storage.

Moreover, according to a method of the present invention, cells are stored (or transported) in an aggregate state, and hence a large number of cells can easily be stored without using a special apparatus or the like. In contrast with this, in the case of storing (or transporting) cells using a conventional freezing method, a large amount of dry ice, a liquid nitrogen tank for transportation or the like is necessary, and hence the work is cumbersome. In the case of storing (or transporting) cells that have been subjected to monolayer culture in a culture vessel such as a flask, a special apparatus or the like is not required since storage is carried out at normal temperature, but the number of cells that can be transported is limited by the area of the vessel walls, and hence the efficiency of transportation is poor.

Furthermore, according to another preferable embodiment of the present invention, there are provided a cell culture method according to which 3-dimensionally reconstructed cells can be cultured easily while maintaining the functions of the cells, and a cell culture obtained using this method.

If cells are cultured in the state of a cell group, which is a collection of cells aggregated at a density of at least approximately 10⁵ cells/ml, then the cells can be cultured over a prolonged period while maintaining the functions of the cells. However, if cells are put into the state of such a cell group, then it becomes difficult to reliably supply nutrients and oxygen as far as the cells on the inside of the cell group. With the method of the present invention, such cell groups are formed adhered to permeable membranes, and a culture solution that contacts the cell groups via the permeable membranes is made to flow around by moving the vessel. As a result, nutrients can be made to reliably reach every part of each cell group easily and efficiently. That is, according to the method of the present invention, cells can be cultivated easily in this way while maintaining the functions of the cells over a prolonged period.

According to another preferable embodiment of the present invention, there are provided a cell differentiation inducing method according to which undifferentiated cells can be induced to differentiate in a fixed direction efficiently, a uniformly differentiated cell culture or tissue body, and a method of transplanting this cell culture or tissue body into a living body.

In the case of culturing undifferentiated cells using a conventional monolayer culture method, it is difficult to induce the whole of the cell group to differentiate in a fixed direction, i.e. some of the cells may differentiate while some remain undifferentiated, or a mixture of a plurality of types of cells having different functions to one another may be produced, or the cells may not differentiate normally. Regarding this point, with the method of the present invention, centrifugal force or pressure is applied to the undifferentiated cells to form cell aggregates on permeable membranes, and then the aggregates are cultured, whereby the whole of the undifferentiated cell group can be made to differentiate uniformly in a fixed direction efficiently.

Furthermore, by adding components that induce cell differentiation to the aggregate culture environment, differentiation can be induced to occur in a fixed direction yet more efficiently.

Moreover, according to the method of the present invention, cell tissue bodies having a 3-dimensional structure and having homogeneous differentiated functions can be obtained easily and efficiently.

Furthermore, according to another preferable embodiment of the present invention, there are provided a cell seeding method according to which cell functions are not prone to dropping during culturing, a cell culture method according to which cell functions can be maintained over a prolonged period, and a cell culture according to which in vivo cell functions are maintained at a high level.

By applying centrifugal force to form aggregates each constituted from a plurality of layers of cells, and thus create a state in which the frequency of contact between cells is greatly increased, cell aggregates in which the functions originally possessed by the cells are exhibited well can be obtained. When culturing these cell aggregates, even if the culturing is carried out over a prolonged period, a drop in the functions of the cells is less prone to occurring than in the case of ordinary monolayer culture or the like.

In the present invention, the aggregates are formed in a layered form on the inner walls of a vessel or on permeable membranes. According to this method, a large number of aggregates can be obtained easily using a widely-used vessel (a multi-well plate or the like), an instrument (a culture insert or the like), and a widely-used apparatus (a centrifuge). Moreover, the centrifugal force can be applied widely and uniformly, and hence by increasing the area of the vessel or the permeable membranes, aggregates comprising a large number of cells can be obtained.

Moreover, such aggregates can easily be induced into tissue bodies, with the tissue bodies formed from the cell aggregates exhibiting the functions originally possessed by the cells well.

Moreover, in the cell culture method of the present invention, by applying the centrifugal force to the cells in a state in which a permeable vessel and a culture vessel have been combined, when forming the aggregates on the permeable membranes of the permeable vessel, it is possible to then fill a cell culture solution into the culture vessel and carry out culturing as is, and hence the culturing of the aggregates can be carried out easily.

In particular, by using a culture insert having a plurality of wells as the permeable vessel, and a multi-well plate having a plurality of wells as the culture vessel, a plurality of cell aggregates can easily be obtained simultaneously, and hence a plurality of types of cell culture can easily be obtained at once without using a special apparatus or the like.

Furthermore, according to another preferable embodiment of the present invention, there is provided a culture method according to which the differentiated functions of cells can be exhibited to a high degree, and moreover the high degree of exhibition can be maintained for a prolonged period.

By applying pressure or the like to form cell aggregates, and thus create a state in which the frequency of contact between cells is greatly increased, cell aggregates in which the functions originally possessed by the cells are exhibited well can be obtained. Moreover, such aggregates can easily be induced into tissue bodies, with the tissue bodies formed from the cell aggregates exhibiting the functions originally possessed by the cells well.

With such cell aggregates or tissue bodies, even if culturing is carried out for a prolonged period, the differentiated functions of the cells are not prone to being lost, with the functions of the cells being maintained well.

Consequently, the cell culture obtained by culturing the cell aggregates is suitable for use as cells for a medical biomaterial.

Moreover, according to the method of the present invention, the pressure can be applied to the cells easily using an injection syringe or the like, and hence a cell culture for which the functions that were possessed by the cells in vivo are exhibited well can be obtained easily.

The present invention further relates to the following inventions.
Item 1B: A method for storing cells, comprising applying centrifugal force to cells to form cell aggregates, and then storing the cells in the aggregate state.
Item 2B: The method according to item 1B, wherein the cells are put into the lumens of hollow fibers each comprising a permeable membrane, and the centrifugal force is applied to the cells in this state to form a cell aggregate in the lumen of each of the hollow fibers.
Item 3B: The method according to item 1B, wherein the cells are put into gaps between the shell and the bundle of hollow fibers of a structure comprising a bundle of hollow fibers each comprising a permeable membrane and a shell enveloping the bundle of hollow fibers, and the centrifugal force is applied to the cells in this state to form cell aggregates in the gaps between the shell and the bundle of hollow fibers.
Item 4B: The method according to item 1B, wherein the cells are placed on permeable membranes, and the centrifugal force is applied to the cells in this state to form a cell aggregate or aggregates on the surface of each of the permeable membranes.
Item 5B: The method according to item 1B, wherein the cells are put into the wells of a vessel having one or a plurality of wells, and the centrifugal force is applied to the cells in this state to form a cell aggregate or aggregates on a bottom part of each well.
Item 6B: A method for storing cells comprising the steps of:
applying pressure to cells to form aggregates; and
storing the cells in the aggregate state.
Item 7B: The method according to item 6B, wherein the cells are put into the lumens of hollow fibers each comprising a permeable membrane, and the pressure is applied to the cells in this state to form a cell aggregate in the lumen of each of the hollow fibers.
Item 8B: The method according to item 6B, wherein the cells are put into gaps between the shell and the bundle of hollow fibers of a structure comprising a bundle of hollow fibers each comprising a permeable membrane and a shell enveloping the bundle of hollow fibers, and the pressure is applied to the cells in this state to form cell aggregates in the gaps between the shell and the bundle of hollow fibers.
Item 9B: The method according to item 6B, wherein the cells are placed on permeable membranes, and the pressure is applied to the cells in this state to form a cell aggregate or aggregates on the surface of each of the permeable membranes.
Item 10B: The method according to item 6B, wherein the cells are put into the wells of a vessel having one or a plurality of wells, and the pressure is applied to the cells in this state to form a cell aggregate or aggregates on a bottom part of each well.
Item 11B: The method according to any of items 1B through 10B, wherein the cells are stored by being placed in a liquid medium for cell culture or a solution for organ transportation.
Item 12B: The method according to any of items 1B through 10B, wherein the cells are stored in a state enveloped by a gel containing a liquid medium for cell culture or a solution for organ transportation.
Item 13B: The method according to any of items 1B through 12B, wherein the cell aggregates are cultured in a culture medium for cell culture to form tissue bodies, and then the cells are stored in the tissue body state.
Item 14B: The method according to item 13B, wherein the cells are stored using the same liquid medium for cell culture as that used when forming the tissue bodies.
Item 15B: The method according to any of items 1B through 14B, wherein the stored cells are hepatocytes.
Item 16B: The method according to any of items 1B through 15B, wherein the stored cells are cells for an artificial organ.
Item 1C: A method for culturing cells comprising the steps of:
adhering a cell group comprising one or a plurality of types of cells on permeable membranes;
making the surface of each of the permeable membranes on which the cell group is not adhered come into contact with a culture solution in a vessel; and
culturing the cells in this state while moving the vessel continuously or intermittently.
Item 2C: The method according to item 1C, wherein each of the permeable membranes takes the form of a hollow fiber.
Item 3C: The method according to item 1C, wherein each of the cell groups forms an aggregate.
Item 4C: The method according to item 3C, wherein the aggregates are formed by applying centrifugal force or pressure to the cells.
Item 5C: The method according to any of items 1C through 4C, wherein each of the cell groups is contained in a gel.
Item 6C: The method according to any of items 1C through 5C, wherein the cells are cells originating from a liver.
Item 7C: The method according to any of items 1C through 6C, wherein the vessel is rotated or moved back and forth in a horizontal direction.

Item 1D: A method for inducing cell differentiation comprising the steps of:
applying centrifugal force or pressure to the undifferentiated cells in a state in which one or a plurality of types of undifferentiated cells have been put into the lumens of hollow fibers each comprising a permeable membrane to form cell aggregates; and
culturing the cell aggregates to induce differentiation of the undifferentiated cells.

Item 2D: A method for inducing cell differentiation comprising the steps of:
applying centrifugal force or pressure to the undifferentiated cells in a state in which one or a plurality of types of undifferentiated cells have been put into gaps between the shell and the hollow fibers of a structure comprising a bundle of hollow fibers each comprising a permeable membrane and a shell enveloping the bundle of hollow fibers to form cell aggregates; and
culturing the cell aggregates to induce differentiation of the undifferentiated cells.
Item 3D: : A method for inducing cell differentiation comprising the steps of:
applying centrifugal force or pressure to the undifferentiated cells in a state in which one or a plurality of types of undifferentiated cells have been placed on the permeable membranes of a vessel having permeable membranes therein to form cell aggregates; and
culturing the cell aggregates to induce differentiation of the undifferentiated cells.
Item 4D: The method according to any of items 1D 2D and 3D, wherein, in the step of culturing the aggregates, the cell aggregates are cultured together with cell differentiation-inducing components.

Item 5D: The method according to any of items 1D, 2D and 3D, wherein, in the step of culturing the aggregates, the cell aggregates are transplanted into a living human or animal, and then cultured in this state.

Item 6D: The method according to any of items 1D through 5D, wherein, in the step to form aggregates, a plurality of cell aggregates for which the type of the cells is the same or mutually different are formed, and in the step of culturing the aggregates, these cell aggregates are cocultured in the same culture system.

Item 7D: The method according to any of items 1D through 6D, wherein the undifferentiated cells are cells selected from the group consisting of non-human embryonic stem cells, ectodermal stem cells, mesodermal stem cells, endodermal stem cells, mesenchymal stem cells, hematopoietic stem cells, neural stem cells, hepatic stem cells, muscle stem cells, pancreatic stem cells, cutaneous stem cells, retinal stem cells, follicular stem cells, bone precursor cells, fat precursor cells, cartilage cells, hair matrix cells, epithelial cells, vascular endothelial cells, smooth muscle cells, cancer cells, and cells in the differentiation lineage from these cells.
Item 1E: A method for seeding cells comprising applying centrifugal force to cells that have been put into a vessel to form cell aggregates each comprising a plurality of layers of the cells on inner walls of the vessel.
Item 2E: The method according to item 1E, wherein the vessel has a plurality of wells, and the centrifugal force is applied to the cells in a state in which the cells have been put into one or a plurality of the wells.
Item 3E: The method according to item 1E or 2E, wherein the cells are hepatocytes.
Item 4E: A method for seeding cells comprising applying centrifugal force to the cells in a state in which cells have been placed on the permeable membranes of a vessel having permeable membranes therein to form cell aggregates each comprising a plurality of layers of the cells on the permeable membranes.
Item 5E: The method according to item 4E, wherein the vessel has a plurality of wells each having a permeable membrane therein, and the centrifugal force is applied to the cells in a state in which the cells have been placed on the permeable membrane in one or a plurality of the wells.
Item 6E: The method according to item 4E or 5E, wherein the cells are hepatocytes.

Item 8E: A method for culturing cells comprising the steps of:
applying centrifugal force to the cells in a state in which cells have been placed on the permeable membranes in a permeable vessel in which the whole or part of a bottom surface thereof is constituted from permeable membranes to form cell aggregates each comprising a plurality of layers of the cells on the permeable membranes; and
culturing the aggregates in a state in which the permeable vessel has been fitted into a culture vessel having a size such that the permeable vessel can be fitted therein. Item 9E: The method according to item 8E, wherein in the step to form aggregates, the centrifugal force is applied to the cells in a state in which the permeable vessel has been fitted into the culture vessel.

Item 10E: The method according to item 8E or 9E, wherein the cells are hepatocytes.
Item 11E: A method for culturing cells comprising the steps of:
applying centrifugal force to the cells in a state in which cells have been placed on one or a plurality of the permeable membranes of a vessel having a plurality of permeable wells each of which has the whole or part of a bottom surface thereof constituted from a permeable membrane to form aggregates each comprising a plurality of layers of the cells on the permeable membranes; and
culturing the aggregates in a state in which the permeable wells have been fitted into wells of a culture vessel having a plurality of wells having a size such that the permeable wells can be fitted therein.
Item 12E: The method according to item 11E, wherein in the step to form aggregates, the centrifugal force is applied to the cells in a state in which the permeable wells have been fitted into the wells of the culture vessel.
Item 13E: The method according to item 11E or 12E, wherein the cells are hepatocytes.
Item 1F: A method for culturing cells comprising the steps of:
applying pressure to cells to form aggregates; and
culturing the cells in the aggregate state.
Item 2F: The method according to item 1F, wherein the cells are put into the lumens of hollow fibers each comprising a permeable membrane, and the pressure is applied to the cells in this state to form a cell aggregate in the lumen of each of the hollow fibers.
Item 3F: The method according to item 1F, wherein the cells are put into gaps between the shell and the bundle of hollow fibers of a structure comprising a bundle of hollow fibers each comprising a permeable membrane and a shell enveloping the bundle of hollow fibers, and the pressure is applied to the cells in this state to form cell aggregates in the gaps between the shell and the bundle of hollow fibers.
Item 4F: The method according to item IF, wherein the cells are placed on permeable membranes, and the pressure is applied to the cells in this state to form a cell aggregate on the surface of each of the permeable membranes.
Item 5F: The method according to item 1F, wherein the cells are put into the wells of a vessel having one or a plurality of wells, and the pressure is applied to the cells in this state to form a cell aggregate on a bottom part of each well.
Item 6F: The method according to any of items 1F through 5F, wherein cells originating from at least one type of tissue selected from the group consisting of cartilage, bone, skin, nerve tissue, oral tissue, alimentary canal, liver, pancreas, kidney, glandular tissue, adrenal, heart, muscle, tendon, fat tissue, connective tissue, reproductive organ, eyeball, blood vessel, bone marrow and blood are used as the cells.
Item 7F: The method according to any of items 1F through 5F, wherein cells of at least one type selected from the group consisting of cartilage cells, osteoblasts, epidermal keratinocytes, melanocytes, nerve cells, neural stem cells, gliacytes, hepatocytes, intestinal epithelial cells, pancreatic beta cells, pancreatic exocrine cells, renal glomerular endothelial cells, tubular epithelial cells, mammary gland cells, thyroid gland cells, salivary gland cells, adrenocortical cells, adrenomedullary cells, myocardial cells, skeletal muscle cells, smooth muscle cells, fat cells, fat precursor cells, lens cells, corneal cells, vascular endothelial cells, bone marrow stromal cells, and lymphocytes are used as the cells.
Item 8F: A cell culture obtained using the method according to any of items 1F through 7F.
Item 9F: A medical biomaterial using the cell culture according to item 8F.

## Claims

1. A hollow fiber-possessing instrument, comprising:
a cell suspension flow tube (1) having one end open;
a hollow fiber fixing part (3) provided in the other end of the cell suspension flow tube (1); and
one or a plurality of hollow fibers (4) that each has one end sealed and the other end open, and each passes through the hollow fiber fixing part (3) such that the open end of the hollow fiber (4) communicates with the cell suspension flow tube (1) and liquid does not leak.

2. The instrument according to claim 1, wherein the open end of the cell suspension flow tube (1) has a shape so as to be fittable to a discharge port of a cell injecting instrument.

3. The instrument according to claim 1, wherein the cell suspension flow tube (1) comprises an inflow side flow tube (11) and an outflow side flow tube (12) that are detachably coupled together.

4. The instrument according to claim 1, further comprising a centrifuging vessel (6) inside which the hollow fibers (4) can be disposed, wherein a lid (62) of the centrifuging vessel (6) is supported by the cell suspension flow tube (1).

5. The instrument according to claim 3, further comprising a centrifuging vessel (6) inside which the hollow fibers (4) can be disposed, wherein a lid (62) of the centrifuging vessel (6) is supported by the inflow side flow tube (11) of the cell suspension flow tube (1).

6. A method of using the hollow fiber-possessing instrument according to any one of claims 1 to 5, comprising injecting a cell suspension from the open end of the cell suspension flow tube (1) of the hollow fiber-possessing instrument to accumulate cells in a lumen of each of the hollow fiber (4) or fibers (4), and culturing or storing the cells in a state in which the hollow fibers (4) having the cells accumulated therein are immersed in a cell culture solution or a solution for cell storage, provided that the cells are not human embryonic cells.

7. The method according to claim 6, wherein the hollow fibers (4) having the cells accumulated in the lumens thereof are separated from the other parts of the instrument, and then the cells are cultured or stored in a state in which the separated hollow fibers (4) are immersed in a cell culture solution or a solution for cell storage.

8. A method of using the hollow fiber-possessing instrument according to claim 4, comprising the steps of: injecting a cell suspension from the open end of the cell suspension flow tube (1) of the hollow fiber-possessing instrument to accumulate cells in a lumen of each of the hollow fibers (4);
applying a centrifugal force to the cells in the hollow fiber lumens in a state in which the hollow fibers (4) are disposed in the centrifuging vessel (6) to form cell aggregates (9) in the hollow fiber lumens; and
culturing or storing the cells in a state in which the hollow fibers (4) holding the cell aggregates (9) are immersed in a cell culture solution or a solution for cell storage, provided that the cells are not human embryonic cells.

9. The method according to claim 8, wherein the hollow fibers (4) having the cell aggregates (9) formed in the lumens thereof are separated from the other parts of the instrument, and then the cells are cultured or stored in a state in which the separated hollow fibers (4) are immersed in a cell culture solution or a solution for cell storage.

10. A method for seeding cells in which a drop in cell functions is suppressed, comprising the steps of:
(a) putting a cell culture solution having one or more types of cells in a culture medium into a lumen or lumens of a porous hollow fiber (4) or fibers (4); and
(b) applying pressure or centrifugal force to the cells in a vessel to form a cell aggregate in a lumen or lumens of the hollow fiber (4) or fibers (4), which is set in a hollow fiber-possessing instrument described in claim 1, provided that the cells are not human embryonic cells.

11. The method according to claim 10, wherein said hollow fibers (4) have a pore size of approximately 0.1 to 5 µm.

12. The method according to claim 10, wherein said hollow fibers (4) have a thickness of approximately 10 to 200 µm.

13. The method according to claim 10, wherein said hollow fibers (4) have an inside diameter of approximately 20 to 1,000 µm.

14. The method according to claim 10, wherein each of the cell aggregates (9) comprises 2 to 20 layers of the cells lying on top of one another.

15. The method according to claim 10, wherein the centrifugal force is approximately 2 to 2,000xG.

16. The method according to claim 10, wherein the pressure is approximately 0.05 to 50 kg/cm².

17. The method according to claim 10, wherein cells originating from at least one type of tissue selected from the group consisting of cartilage, bone, skin, nerve tissue, oral tissue, alimentary canal, liver, pancreas, kidney, glandular tissue, adrenal, heart, muscle, tendon, fat tissue, connective tissue, reproductive organ tissue, eyeball, blood vessel, bone marrow and blood are used as the cells,

18. The method according to claim 17, wherein cells of at least one type selected from the group consisting of cartilage cells, osteoblasts, epidermal keratinocytes, melanocytes, nerve cells, neural stem cells, gliacytes, hepatocytes, intestinal epithelial cells, pancreatic beta cells, pancreatic exocrine cells, renal glomerular endothelial cells, tubular epithelial cells, mammary gland cells, thyroid gland cells, salivary gland cells, adrenocortical cells, adrenomedullary cells, myocardial cells, skeletal muscle cells, smooth muscle cells, fat cells, fat precursor cells, lens cells, corneal cells, vascular endothelial cells, bone marrow stromal cells, and lymphocytes are used as the cells.

19. The method according to claim 10, wherein the cells are undifferentiated cells selected from the group consisting of non-human embryonic stem cells, ectodermal stem cells, mesodermal stem cells, endodermal stem cells, mesenchymal stem cells, hematopoietic stem cells, neural stem cells, hepatic stem cells, muscle stem cells, pancreatic stem cells, cutaneous stem cells, retinal stem cells, follicular stem cells, bone precursor cells, fat precursor cells, cartilage cells, hair matrix cells, epithelial cells, vascular endothelial cells, smooth muscle cells, cancer cells, and cells in the differentiation lineage from these cells.

20. A method for culturing cells in which a drop in cell functions is suppressed, the method-further comprising a step of culturing the cell aggregates (9) obtained with the method of claim 10.

21. A method for culturing cells comprising bringing the hollow fibers (4) having formed therein the cell aggregates (9) obtained using the method of claim 10 into contact with a culture solution in a vessel, and culturing cells in this state while moving the vessel continuously or intermittently.

22. The method according to claim 21, wherein the cell aggregates are contained in a gel.

23. The method according to claim 21, wherein the cells are cells originating from a liver.

24. The method according to claim 21, wherein the vessel is rotated or moved back and forth in a horizontal direction.

25. A method for inducing cell differentiation, comprising a step of culturing aggregates of undifferentiated cells obtained using the method of claim 10 to induce differentiation of the undifferentiated cells.

26. The method according to claim 25, wherein the cell aggregates (9) are cultured together with cell differentiation-inducing components, to induce differentiation of the undifferentiated cells.

27. The method according to claim 25, wherein a plurality of cell aggregates for which the type of the cells is the same or mutually different are formed, and these cell aggregates (9) are cocultured in the same culture system.

28. A method for storing cells for which a drop in cell functions is suppressed, the method further having a step of storing the cell aggregates obtained with the method of claim 10.

29. The method according to claim 28, wherein the cells are stored by being placed in a liquid medium for cell culture or a solution for organ transportation.

30. The method according to claim 28, wherein the cells are stored in a state enveloped by a gel containing a liquid medium for cell culture or a solution for organ transportation.

31. The method according to claim 28, wherein the stored cells are hepatocytes.

32. The method according to claim 28, wherein the stored cells are cells for an artificial organ.

## Patentansprüche

1. Hohlfaser-aufweisendes Instrument, umfassend:
Zellsuspensions-Durchflussrohr (1) mit einem offenen Ende;
Hohlfaser-Fixierteil (3), das sich im anderen Ende des Zellsuspensions-Durchflussrohrs (1) befindet; und
eine oder mehrere Hohlfasern (4), die jeweils ein geschlossenes und ein offenes Ende aufweisen und jeweils durch den Hohlfaser-Fixierteil (3) hindurch verlaufen, so dass das offene Ende der Hohlfaser (4) mit dem Zellsuspensions-Durchflussrohr (1) in Verbindung steht und keine Flüssigkeit austritt.

2. Instrument gemäß Anspruch 1, wobei das offene Ende des Zellsuspensions-Durchflussrohrs (1) eine solche Form hat, dass es sich an einen Entleerungsanschluss eines Zellinjektionsinstruments anschließen lässt.

3. Instrument gemäß Anspruch 1, wobei das Zellsuspensions-Durchflussrohr (1) ein Einfließseiten-Durchflussrohr (11) und ein Ausfließseiten-Durchflussrohr (12) umfasst, die lösbar aneinander gekoppelt sind.

4. Instrument gemäß Anspruch 1, das weiterhin ein Zentrifugiergefäß (6) umfasst, innerhalb dessen die Hohlfasern (4) angeordnet sein können, wobei ein Deckel (62) des Zentrifugiergefäßes (6) von dem Zellsuspensions-Durchflussrohr (1) gehalten wird.

5. Instrument gemäß Anspruch 3, das weiterhin ein Zentrifugiergefäß (6) umfasst, innerhalb dessen die Hohlfasern (4) angeordnet sein können, wobei ein Deckel (62) des Zentrifugiergefäßes (6) von dem Einfließseiten-Durchflussrohr (12) des Zellsuspensions-Durchflussrohrs (1) gehalten wird.

6. Verfahren zur Verwendung des Hohlfaser-aufweisenden Instruments gemäß einem der Ansprüche 1 bis 5, umfassend das Injizieren einer Zellsuspension vom offenen Ende des Zellsuspensions-Durchflussrohrs (1) des Hohlfaser-aufweisenden Instruments her, so dass Zellen in einem Lumen von jedem der Hohlfaser(n) (4) angesammelt werden, und Kultivieren oder Aufbewahren der Zellen in einem Zustand, in dem die Hohlfasern (4) mit den darin angesammelten Zellen in eine Zellkulturlösung oder eine Lösung zur Zellaufbewahrung eingetaucht sind, mit der Maßgabe, dass die Zellen keine humanen embryonalen Zellen sind.

7. Verfahren gemäß Anspruch 6, wobei die Hohlfasern (4) mit den in ihren Lumen angesammelten Zellen von den anderen Teilen des Instruments getrennt werden und dann die Zellen in einem Zustand, in dem die abgetrennten Hohlfasern (4) in eine Zellkulturlösung oder eine Lösung zur Zellaufbewahrung eingetaucht sind, kultiviert oder aufbewahrt werden.

8. Verfahren zur Verwendung des Hohlfaser-aufweisenden Instruments gemäß Anspruch 4, das die folgenden Schritte umfasst: Injizieren einer Zellsuspension vom offenen Ende des Zellsuspensions-Durchflussrohrs (1) des Hohlfaser-aufweisenden Instruments her, so dass Zellen in einem Lumen von jedem der Hohlfasern (4) angesammelt werden;
Ausüben einer Zentrifugalkraft auf die Zellen in den Hohlfaserlumen in einem Zustand, in dem die Hohlfasern (4) in dem Zentrifugiergefäß (6) angeordnet sind, unter Bildung von Zellaggregaten (9) in den Hohlfaserlumen; und
Kultivieren oder Aufbewahren der Zellen in einem Zustand, in dem die Hohlfasern (4), die die Zellaggregate (9) enthalten, in eine Zellkulturlösung oder eine Lösung zur Zellaufbewahrung eingetaucht sind, mit der Maßgabe, dass die Zellen keine humanen embryonalen Zellen sind.

9. Verfahren gemäß Anspruch 8, wobei die Hohlfasern (4) mit den in ihren Lumen gebildeten Zellaggregaten (9) von den anderen Teilen des Instruments getrennt werden und dann die Zellen in einem Zustand, in dem die abgetrennten Hohlfasern (4) in eine Zellkulturlösung oder eine Lösung zur Zellaufbewahrung eingetaucht sind, kultiviert oder aufbewahrt werden.

10. Verfahren zum Aussäen von Zellen, wobei ein Abfall der Zellfunktionen unterdrückt wird, umfassend die folgenden Schritte:
(a) Einbringen einer Zellkulturlösung, die ein oder mehrere Typen von Zellen in einem Kulturmedium aufweist, in ein oder mehrere Lumen einer oder mehrerer porösen Hohlfasern (4); und
(b) Ausüben von Druck oder einer Zentrifugalkraft auf die Zellen in einem Gefäß unter Bildung eines Zellaggregats in einem oder mehreren Lumen der Hohlfaser oder -fasern (4), das in einem Hohlfaser-aufweisenden Instrument, wie es in Anspruch 1 beschrieben ist, angeordnet ist, mit der Maßgabe, dass die Zellen keine humanen embryonalen Zellen sind.

11. Verfahren gemäß Anspruch 10, wobei die Hohlfasern (4) eine Porengröße von ungefähr 0,1 bis 5 µm haben.

12. Verfahren gemäß Anspruch 10, wobei die Hohlfasern (4) eine Dicke von ungefähr 10 bis 200 µm haben.

13. Verfahren gemäß Anspruch 10, wobei die Hohlfasern (4) einen Innendurchmesser von ungefähr 20 bis 1000 µm haben.

14. Verfahren gemäß Anspruch 10, wobei jedes der Zellaggregate (9) 2 bis 20 Schichten der Zellen umfasst, die übereinander liegen.

15. Verfahren gemäß Anspruch 10, wobei die Zentrifugalkraft ungefähr 2 bis 2000 x g beträgt.

16. Verfahren gemäß Anspruch 10, wobei der Druck ungefähr 0,05 bis 50 kg/cm² beträgt.

17. Verfahren gemäß Anspruch 10, wobei Zellen, die aus wenigstens einer Art von Gewebe stammen, das aus der Gruppe ausgewählt ist, die aus Knorpel, Knochen, Haut, Nervengewebe, Mundgewebe, Nahrungskanal, Leber, Pankreas, Niere, Drüsengewebe, Nebenniere, Herz, Muskel, Sehne, Fettgewebe, Bindegewebe, Reproduktionsorgangewebe, Augapfel, Blutgefäß, Knochenmark und Blut besteht, als Zellen verwendet werden.

18. Verfahren gemäß Anspruch 17, wobei Zellen wenigstens einer Art, die aus der Gruppe ausgewählt ist, die aus Knorpelzellen, Osteoblasten, epidermalen Keratinocyten, Melanocyten, Nervenzellen, neuralen Stammzellen, Gliacyten, Hepatocyten, Darmepithelzellen, Pankreas-β-Zellen, exokrinen Pankreaszellen, Nierenglomerulum-Endothelzellen, Tubulus-Epithelzellen, Milchdrüsenzellen, Schilddrüsenzellen, Speicheldrüsenzellen, Nebennierenrindenzellen, Nebennierenmarkzellen, Myokardzellen, Skelettmuskelzellen, Zellen der glatten Muskulatur, Fettzellen, Fettvorläuferzellen, Linsenzellen, Korneazellen, Gefäßendothelzellen, Knochenmarkstromazellen und Lymphocyten besteht, als Zellen verwendet werden.

19. Verfahren gemäß Anspruch 10, wobei die Zellen undifferenzierte Zellen sind, die aus der Gruppe ausgewählt sind, die aus nichthumanen embryonalen Stammzellen, ektodermalen Stammzellen, mesodermalen Stammzellen, endodermalen Stammzellen, mesenchymalen Stammzellen, hämatopoetischen Stammzellen, neuralen Stammzellen, hepatischen Stammzellen, Muskelstammzellen, Pankreasstammzellen, Hautstammzellen, Retinastammzellen, Follikelstammzellen, Knochenvorläuferzellen, Fettvorläuferzellen, Knorpelzellen, Haarmatrixzellen, Epithelzellen, Gefäßendothelzellen, Zellen der glatten Muskulatur, Krebszellen und Zellen in der von diesen Zellen ausgehenden Differenzierungslinie besteht.

20. Verfahren zum Kultivieren von Zellen, wobei ein Abfall der Zellfunktionen unterdrückt wird, wobei das Verfahren weiterhin einen Schritt des Kultivierens der Zellaggregate (9) umfasst, die mit dem Verfahren von Anspruch 10 erhalten werden.

21. Verfahren zum Kultivieren von Zellen, umfassend das In-Kontakt-Bringen der Hohlfasern (4) mit den darin gebildeten Zellaggregaten (9), die mit Hilfe des Verfahrens von Anspruch 10 erhalten wurden, mit einer Kulturlösung in einem Gefäß und das Kultivieren der Zellen in diesem Zustand, während das Gefäß ständig oder mit Unterbrechungen bewegt wird.

22. Verfahren gemäß Anspruch 21, wobei die Zellaggregate in einem Gel enthalten sind.

23. Verfahren gemäß Anspruch 21, wobei die Zellen aus einer Leber stammende Zellen sind.

24. Verfahren gemäß Anspruch 21, wobei das Gefäß rotieren gelassen oder in horizontaler Richtung vor- und zurückbewegt wird.

25. Verfahren zum Induzieren der Zelldifferenzierung, das einen Schritt des Kultivierens von Aggregaten von undifferenzierten Zellen, die mit Hilfe des Verfahrens von Anspruch 10 erhalten wurden, in einer solchen Weise umfasst, dass die Differenzierung der undifferenzierten Zellen induziert wird.

26. Verfahren gemäß Anspruch 25, wobei die Zellaggregate (9) zusammen mit die Zelldifferenzierung induzierenden Komponenten kultiviert werden, so dass die Differenzierung der undifferenzierten Zellen induziert wird.

27. Verfahren gemäß Anspruch 25, wobei mehrere Zellaggregate, bei denen die Art der Zellen gleich oder jeweils unterschiedlich ist, gebildet werden und diese Zellaggregate (9) in demselben Kultursystem cokultiviert werden.

28. Verfahren zur Aufbewahrung von Zellen, bei denen ein Abfall der Zeilfunktionen unterdrückt wird, wobei das Verfahren weiterhin einen Schritt des Aufbewahrens der Zellaggregate (9) umfasst, die mit dem Verfahren von Anspruch 10 erhalten werden.

29. Verfahren gemäß Anspruch 28, wobei die Zellen aufbewahrt werden, indem man sie in ein flüssiges Medium für die Zellkultur oder eine Lösung für den Organtransport gibt.

30. Verfahren gemäß Anspruch 28, wobei die Zellen in einem Zustand aufbewahrt werden, in dem sie mit einem Gel umwickelt sind, das ein flüssiges Medium für die Zellkultur oder eine Lösung für den Organtransport enthält.

31. Verfahren gemäß Anspruch 28, wobei die aufbewahrten Zellen Hepatocyten sind.

32. Verfahren gemäß Anspruch 28, wobei die aufbewahrten Zellen Zellen für ein künstliches Organ sind.

## Revendications

1. Instrument à fibres creuses, comprenant :
◆ un tube d'écoulement de cellules en suspension (1) ayant une extrémité ouverte ;
◆ une partie de fixation de fibres creuses (3) prévue à l'autre extrémité du tube d'écoulement de cellules en suspension (1) ; et
◆ une ou une pluralité de fibre(s) creuse(s) (4) qui possèdent chacune une extrémité scellée et l'autre extrémité ouverte, et passent chacune à travers la partie de fixation de fibres creuses (3) de telle sorte que l'extrémité ouverte de la fibre creuse (4) communique avec le tube d'écoulement de cellules en suspension (1) et que le liquide ne fuit pas.

2. Instrument selon la revendication 1, dans lequel l'extrémité ouverte du tube d'écoulement de cellules en suspension (1) a une forme adaptable à l'orifice de sortie d'un instrument d'injection de cellules.

3. Instrument selon la revendication 1, dans lequel le tube d'écoulement de cellules en suspension (1) comprend un tube d'écoulement du côté entrant (11) et un tube d'écoulement du côté sortant (12) qui sont raccordés l'un à l'autre de façon détachable.

4. Instrument selon la revendication 1, comprenant en outre une cuve centrifugeuse (6) à l'intérieur de laquelle les fibres creuses (4) peuvent être disposées, un couvercle (62) de la cuve centrifugeuse (6) étant supporté par le tube d'écoulement de cellules en suspension (1).

5. Instrument selon la revendication 3, comprenant en outre une cuve centrifugeuse (6) à l'intérieur de laquelle les fibres creuses (4) peuvent être disposées, un couvercle (62) de la cuve centrifugeuse (6) étant supporté par le tube d'écoulement du côté entrant (11) du tube d'écoulement de cellules en suspension (1).

6. Méthode utilisant l'instrument à fibres creuses selon l'une quelconque des revendications 1 à 5, comprenant l'injection de cellules en suspension depuis l'extrémité ouverte du tube d'écoulement de cellules en suspension (1) de l'instrument à fibres creuses pour accumuler les cellules dans un lumen de chacune de la fibre creuse (4) ou des fibres creuses (4), et la culture ou la conservation des cellules dans un état où les fibres creuses (4) avec les cellules accumulées à l'intérieur sont immergées dans une solution de culture de cellules ou une solution de conservation de cellules, à condition que les cellules ne soient pas des cellules embryonnaires humaines.

7. Méthode selon la revendication 6, dans laquelle les fibres creuses (4) dont les cellules s'accumulent dans les lumens de celles-ci sont séparées des autres parties de l'instrument, puis les cellules sont cultivées ou conservées dans un état où les fibres creuses séparées (4) sont immergées dans une solution de culture de cellules ou une solution de conservation de cellules.

8. Méthode utilisant l'instrument à fibres creuses selon la revendication 4, comprenant les étapes consistant à :
◆ injecter des cellules en suspension depuis l'extrémité ouverte du tube d'écoulement de cellules en suspension (1) de l'instrument à fibres creuses pour accumuler les cellules dans un lumen de chacune des fibres creuses (4) ;
◆ exercer une force centrifuge sur les cellules dans le lumen des fibres creuses, dans un état où les fibres creuses (4) sont disposées dans la cuve centrifugeuse (6) pour former des agrégats cellulaires (9) dans le lumen des fibres creuses ; et
◆ cultiver ou conserver les cellules dans un état où les fibres creuses (4) contenant les agrégats cellulaires (9) sont immergées dans une solution de culture de cellules ou une solution de conservation de cellules, à condition que les cellules ne soient pas des cellules embryonnaires humaines.

9. Méthode selon la revendication 8, dans laquelle les fibres creuses (4) dont les agrégats cellulaires (9) sont formés (9) dans le lumen de celles-ci sont séparées des autres parties de l'instrument, puis les cellules sont cultivées ou conservées dans un état où les fibres creuses séparées (4) sont immergées dans une solution de culture de cellules ou une solution de conservation de cellules.

10. Méthode d'ensemencement de cellules dans laquelle une perte des fonctions des cellules est évitée, comprenant les étapes consistant à :
(a) placer une solution de culture de cellules comportant un ou plusieurs types de cellules dans milieu de culture à l'intérieur d'un lumen ou de lumens d'une ou de fibre (s) creuse (s) poreuse (s) (4) ; et
(b) exercer une pression ou une force centrifuge sur les cellules dans une cuve pour former un agrégat de cellules dans un lumen ou des lumens de la ou des fibre(s) creuse(s) (4), qui est placé dans l'instrument à fibres creuses décrit dans la revendication 1, à condition que les cellules ne soient pas des cellules embryonnaires humaines.

11. Méthode selon la revendication 10, dans laquelle lesdites fibres creuses (4) présentent un diamètre de pore d'environ 0,1 à 5 µm.

12. Méthode selon la revendication 10, dans laquelle lesdites fibres creuses (4) présentent une épaisseur d'environ 10 à 200 µm.

13. Méthode selon la revendication 10, dans laquelle lesdites fibres creuses (4) présentent un diamètre interne d'environ 20 à 1000 µm.

14. Méthode selon la revendication 10, dans laquelle chacun des agrégats de cellules (9) comprend 2 à 20 couches de cellules superposées les unes aux autres.

15. Méthode selon la revendication 10, dans laquelle la force centrifuge est d'environ 2 à 2000xG.

16. Méthode selon la revendication 10, dans laquelle la pression est d'environ 0,05 à 50 kg/cm².

17. Méthode selon la revendication 10, dans laquelle les cellules issues d'au moins un type de tissu sélectionné dans le groupe comprenant le cartilage, l'os, la peau, le tissu nerveux, le tissu oral, le canal alimentaire, le foie, le pancréas, les reins, le tissu glandulaire, le surrénal, le coeur, les muscles, les tendons, le tissu adipeux, le tissu conjonctif, le tissu des organes reproducteurs, le globe oculaire, les vaisseaux sanguins, la moelle osseuse et le sang, sont utilisées comme cellules.

18. Méthode selon la revendication 17, dans laquelle les cellules d'au moins un type sélectionné dans le groupe comprenant les cellules cartilagineuses, les ostéoblastes, les kératinocytes épidermiques, les mélanocytes, les cellules nerveuses, les cellules souches neuronales, les gliacytes, les hépatocytes, les cellules épithéliales intestinales, les cellules β pancréatiques, les cellules exocrines pancréatiques, les cellules endothéliales glomérulaires rénales, les cellules épithéliales tubulaires, les cellules des glandes mammaires, les cellules des glandes thyroïdes, les cellules des glandes salivaires, les cellules corticosurrénales, les cellules adrenomédullaires, les cellules myocardiques, les cellules des muscles squelettiques, les cellules des muscles viscéraux, les cellules adipeuses, les cellules précurseuses adipeuses, les cellules du cristallin, les cellules de la cornée, les cellules endothéliales vasculaires, les cellules du stroma de la moelle osseuse, et les lymphocytes sont utilisées comme cellules.

19. Méthode selon la revendication 10, dans laquelle les cellules sont des cellules indifférenciées sélectionnées dans le groupe comprenant les cellules souches embryonnaires non humaines, les cellules souches ectodermiques, les cellules souches mésodermiques, les cellules souches endodermiques, les cellules souches mésenchymateuses, les cellules souches hématopoïétiques, les cellules souches neuronales, les cellules souches hépatiques, les cellules souches musculaires, les cellules souches pancréatiques, les cellules souches cutanées, les cellules souches rétiniennes, les cellules souches folliculaires, les cellules précurseuses osseuses, les cellules précurseuses adipeuses, les cellules cartilagéneuses, les cellules de la matrice capillaire, les cellules épithéliales, les cellules endothéliales vasculaires, les cellules des muscles viscéraux, les cellules cancéreuses, et les cellules de différenciation de la descendance issues de ces cellules.

20. Méthode de culture de cellules dans laquelle une perte des fonctions des cellules est évitée, cette méthode comprenant en outre une étape de culture des agrégats cellulaires (9) obtenus à l'aide de la méthode selon la revendication 10.

21. Méthode de culture de cellules comprenant la mise en contact des fibres creuses (4) ayant, formées à l'intérieur, les agrégats cellulaires (9) obtenus à l'aide de la méthode selon la revendication 10, avec une solution de culture dans une cuve, et la culture des cellules dans cet état, tout en remuant la cuve de façon continue ou intermittente.

22. Méthode selon la revendication 21, dans laquelle les agrégats cellulaires sont contenus dans un gel.

23. Méthode selon la revendication 21, dans laquelle les cellules sont des cellules provenant du foie.

24. Méthode selon la revendication 21, dans laquelle on fait tourner la cuve ou dans laquelle on la déplace d'avant en arrière dans le sens horizontal.

25. Méthode pour induire une différenciation cellulaire, comprenant l'étape de culture d'agrégats de cellules indifférenciées obtenus à l'aide de la méthode selon la revendication 10, pour induire la différenciation des cellules indifférenciées.

26. Méthode selon la revendication 25, dans laquelle les agrégats cellulaires (9) sont cultivés avec des composants induisant une différenciation cellulaire, pour induire la différenciation des cellules indifférenciées.

27. Méthode selon la revendication 25, dans laquelle une pluralité d'agrégats cellulaires dont les types des cellules sont identiques ou mutuellement différent est formée, et ces agrégats cellulaires (9) sont cultives ensemble dans le même système de culture.

28. Méthode de conservation de cellules dans laquelle la perte des fonctions des cellules est évitée, cette méthode comportant en outre une étape de conservation des agrégats cellulaires obtenus à l'aide de la méthode selon la revendication 10.

29. Méthode selon la revendication 28, dans laquelle on conserve les cellules en les plaçant dans un milieu liquide de culture de cellules ou une solution de transport d'organes.

30. Méthode selon la revendication 28, dans laquelle on conserve les cellules dans un état où elles sont enveloppées par un gel contenant un milieu liquide de culture de cellules ou une solution de transport d'organes.

31. Méthode selon la revendication 28, dans laquelle les cellules conservées sont des hépatocytes.

32. Méthode selon la revendication 28, dans laquelle les cellules conservées sont des cellules pour un organe artificiel.
